# EUROPEAN PATENT SPECIFICATION

(11) **EP 3 070 163 B1**
(45) Date of publication and mention of the grant of the patent: **06.01.2021**
(21) Application number: 14862407.5
(22) Date of filing: 12.11.2014
(51) Int. Cl.: C12N 5/074, C07K 14/435

(54) **METHOD FOR PREPARING INDUCED PLURIPOTENT STEM CELL, COMPOSITION USED IN METHOD, AND USES THEREOF**
VERFAHREN ZUR HERSTELLUNG INDUZIERTER PLURIPOTENTER STAMMZELLEN, ZUSAMMENSETZUNG HIERFÜR UND VERWENDUNGEN DAVON
PROCÉDÉ DE PRÉPARATION DE CELLULE SOUCHE PLURIPOTENTE INDUITE, COMPOSITION UTILISÉE DANS LE PROCÉDÉ ET UTILISATIONS DU PROCÉDÉ

(30) Priority: 15.11.2013 CN 201310574447
(43) Date of publication of application: 21.09.2016
(73) Proprietor: Guangzhou Institutes of Biomedicine and Health, Chinese Academy of Sciences, Guangzhou, Guangdong 510530 (CN)
(72) Inventor: PEI, Duanqing, Guang Zhou Guangdong 510530 (CN); LIU, Jing, Guang Zhou Guangdong 510530 (CN); CHEN, Jiekai, Guang Zhou Guangdong 510530 (CN); PENG, Meixiu, Guang Zhou Guangdong 510530 (CN)
(74) Representative: Haseltine Lake Kempner LLP
(86) International application number: PCT/CN2014/090882
(87) International publication number: WO 2015/070756

(56) References cited:
- EP-A1- 2 647 700
- WO-A1-2010/000491
- CN-A- 101 864 451
- CN-A- 102 417 894
- CN-A- 102 816 796
- JP-A- 2012 019 784
- US-A1- 2010 190 250
- SHYH-SHIN CHIOU ET AL: "Control of Oxidative Stress and Generation of Induced Pluripotent Stem Cell-like Cells by Jun Dimerization Protein 2", CANCERS, vol. 5, no. 3, 26 July 2013 (2013-07-26), pages 959-984, XP055372058, DOI: 10.3390/cancers5030959
- JING LIU ET AL: "The oncogene c-Jun impedes somatic cell reprogramming", NATURE CELL BIOLOGY, vol. 17, no. 7, 22 June 2015 (2015-06-22), pages 856-867, XP055371052, GB ISSN: 1465-7392, DOI: 10.1038/ncb3193
- DATABASE GENBANK [Online] 29 November 2011 'GLYCOSIDE HYDROLASE FAMILY 3 PROTEIN [TRICHODERMA ATROVIRIDE IMI 206040]', XP055343939 Retrieved from NCBI Database accession no. EHK42488

<110> GUANGZHOU INSTITUTES OF BIOMEDICINE AND HEALTH, CHINESE ACADEMY OF SCIENCES
<120> METHOD FOR PREPARING INDUCED PLURIPOTENT STEM CELL, COMPOSITION USED IN METHOD, AND USES THEREOF
<130> P04-1P
<160> 3
<170> PatentIn version 3.3
<210> 1
   <211> 334
   <212> PRT
   <213> amino acid sequence of c-JunWT of mouse
<400> 1
   Met Thr Ala Lys Met Glu Thr Thr Phe Tyr Asp Asp Ala Leu Asn Ala
   1               5                   10                  15
   Ser Phe Leu Gln Ser Glu Ser Gly Ala Tyr Gly Tyr Ser Asn Pro Lys
               20                  25                  30
   Ile Leu Lys Gln Ser Met Thr Leu Asn Leu Ala Asp Pro Val Gly Ser
           35                  40                  45
   Leu Lys Pro His Leu Arg Ala Lys Asn Ser Asp Leu Leu Thr Ser Pro
       50                  55                  60
   Asp Val Gly Leu Leu Lys Leu Ala Ser Pro Glu Leu Glu Arg Leu Ile
   65                  70                  75                  80
   Ile Gln Ser Ser Asn Gly His Ile Thr Thr Thr Pro Thr Pro Thr Gln
                   85                  90                  95
   Phe Leu Cys Pro Lys Asn Val Thr Asp Glu Gln Glu Gly Phe Ala Glu
               100                 105                 110
   Gly Phe Val Arg Ala Leu Ala Glu Leu His Ser Gln Asn Thr Leu Pro
           115                 120                 125
   Ser Val Thr Ser Ala Ala Gln Pro Val Ser Gly Ala Gly Met Val Ala
       130                 135                 140
   Pro Ala Val Ala Ser Val Ala Gly Ala Gly Gly Gly Gly Gly Tyr Ser
   145                 150                 155                 160
   Ala Ser Leu His Ser Glu Pro Pro Val Tyr Ala Asn Leu Ser Asn Phe
                   165                 170                 175
   Asn Pro Gly Ala Leu Ser Ser Gly Gly Gly Ala Pro Ser Tyr Gly Ala
               180                 185                 190
   Ala Gly Leu Ala Phe Pro Ser Gln Pro Gln Gln Gln Gln Gln Pro Pro
           195                 200                 205
   Gln Pro Pro His His Leu Pro Gln Gln Ile Pro Val Gln His Pro Arg
       210                 215                 220
   Leu Gln Ala Leu Lys Glu Glu Pro Gln Thr Val Pro Glu Met Pro Gly
   225                 230                 235                 240
   Glu Thr Pro Pro Leu Ser Pro Ile Asp Met Glu Ser Gln Glu Arg Ile
                   245                 250                 255
   Lys Ala Glu Arg Lys Arg Met Arg Asn Arg Ile Ala Ala Ser Lys Cys
               260                 265                 270
   Arg Lys Arg Lys Leu Glu Arg Ile Ala Arg Leu Glu Glu Lys Val Lys
           275                 280                 285
   Thr Leu Lys Ala Gln Asn Ser Glu Leu Ala Ser Thr Ala Asn Met Leu
       290                 295                 300
   Arg Glu Gln Val Ala Gln Leu Lys Gln Lys Val Met Asn His Val Asn
   305                 310                 315                 320
   Ser Gly Cys Gln Leu Met Leu Thr Gln Gln Leu Gln Thr Phe
                   325                 330
<210> 2
   <211> 165
   <212> PRT
   <213> amino acid sequence of c-JunDN of mouse
<400> 2
   Tyr Ala Asn Leu Ser Asn Phe Asn Pro Gly Ala Leu Ser Ser Gly Gly
   1               5                   10                  15
   Gly Ala Pro Ser Tyr Gly Ala Ala Gly Leu Ala Phe Pro Ser Gln Pro
               20                  25                  30
   Gln Gln Gln Gln Gln Pro Pro Gln Pro Pro His His Leu Pro Gln Gln
           35                  40                  45
   Ile Pro Val Gln His Pro Arg Leu Gln Ala Leu Lys Glu Glu Pro Gln
       50                  55                  60
   Thr Val Pro Glu Met Pro Gly Glu Thr Pro Pro Leu Ser Pro Ile Asp
   65                  70                  75                  80
   Met Glu Ser Gln Glu Arg Ile Lys Ala Glu Arg Lys Arg Met Arg Asn
                   85                  90                  95
   Arg Ile Ala Ala Ser Lys Cys Arg Lys Arg Lys Leu Glu Arg Ile Ala
               100                 105                 110
   Arg Leu Glu Glu Lys Val Lys Thr Leu Lys Ala Gln Asn Ser Glu Leu
           115                 120                 125
   Ala Ser Thr Ala Asn Met Leu Arg Glu Gln Val Ala Gln Leu Lys Gln
       130                 135                 140
   Lys Val Met Asn His Val Asn Ser Gly Cys Gln Leu Met Leu Thr Gln
   145                 150                 155                 160
   Gln Leu Gln Thr Phe
                   165
<210> 3
   <211> 163
   <212> PRT
   <213> amino acid sequence of Jdp2 of mouse
<400> 3
   Met Met Pro Gly Gln Ile Pro Asp Pro Ser Val Thr Ala Gly Ser Leu
   1               5                   10                  15
   Pro Gly Leu Gly Pro Leu Thr Gly Leu Pro Ser Ser Ala Leu Thr Thr
               20                  25                  30
   Glu Glu Leu Lys Tyr Ala Asp Ile Arg Asn Ile Gly Ala Met Ile Ala
           35                  40                  45
   Pro Leu His Phe Leu Glu Val Lys Leu Gly Lys Arg Pro Gln Pro Val
       50                  55                  60
   Lys Ser Glu Leu Asp Glu Glu Glu Glu Arg Arg Lys Arg Arg Arg Glu
   65                  70                  75                  80
   Lys Asn Lys Val Ala Ala Ala Arg Cys Arg Asn Lys Lys Lys Glu Arg
                   85                  90                  95
   Thr Glu Phe Leu Gln Arg Glu Ser Glu Arg Leu Glu Leu Met Asn Ala
               100                 105                 110
   Glu Leu Lys Thr Gln Ile Glu Glu Leu Lys Leu Glu Arg Gln Gln Leu
           115                 120                 125
   Ile Leu Met Leu Asn Arg His Arg Pro Thr Cys Ile Val Arg Thr Asp
       130                 135                 140
   Ser Val Arg Thr Pro Glu Ser Glu Gly Asn Pro Leu Leu Glu Gln Leu
   145                 150                 155                 160
   Asp Lys Lys

## Description

This application claims the benefit of priority to Chinese Patent Application No. 201310574447.5, filed with the SIPO on November 15, 2013, entitled "METHOD FOR PREPARING INDUCED PLURIPOTENT STEM CELL, COMPOSITION USED IN METHOD, AND USES THEREOF".

### TECHNICAL FIELD

The present invention relates to a method for preparing an induced pluripotent stem cell, a composition for promoting the formation of an induced pluripotent stem cell (iPSC) and uses thereof.

### BACKGROUND ART

Stem cells, a population of cells capable of self-renewal through cell mitosis, can differentiate into various specialized cells under certain conditions. The stem cells' ability to self-renew is used as the basis for cell differentiation and specialization necessary for the development of organs and tissues. Recent evidences show that stem cells are useful for tissue reconstruction and recovery of physiological functions and tissue functions. Therefore, stem cells possess the potential for treating various diseases and damages (including nervous system damage, malignant tumor, hereditary disease, hemoglobin disease and immunodeficiency). Cell transplantation therapy using stem cells is an important aspect of regenerative medicine research. Stem cell transplantation can be useful in the treatment of cardiac injury, nervous system degenerative disease, spinal cord injury, renal failure, disease of blood system and so on. However, cell transplantation therapy faces many difficulties such as allograft rejection, limited cell source and the like.

Induced pluripotent stem cells (iPSCs) are a kind of cells that are similar to embryonic stem cells and have developmental pluripotency. By introducing a certain gene to induce a somatic cell, the somatic cell gains characteristics of a stem cell.

In 2006, Yamanaka's Lab of Kyoto University announced for the first time that mouse fibroblasts were successfully reprogrammed inductively into pluripotent stem cells by introducing four genes (i.e., Oct4, Sox2, c-Myc and Klf4) into mouse fibroblasts and the obtained stem cells exhibited properties similar to embryonic stem cells. In this experiment, Yamanaka et al. firstly selected 24 genes associated with the self-renewal and the maintenance of pluripotency of mouse embryonic stem cells, and then cloned them to retroviral vectors respectively so as to carry out co-transfection of embryonic fibroblasts. After screening on the basis of Fbx15 reporter system, they found the formation of cell colonies of embryonic-like stem cells. Through further analysis of the 24 genes, they found that embryonic fibroblasts can be transformed into induced pluripotent stem cells (iPSCs) completely by simply transducing Oct4, Sox2, c-Myc and Klf4. The induced pluripotent stem cells had normal karyotypes, expressed molecular markers similar to embryonic stem cells and can be inductively differentiated into differentiated cells of three germ layers (i.e., inner, middle and outer layers) in vitro. However, the iPSCs obtained from this experiment were different from embryonic stem cells in terms of gene expression and methylation mode, and no living chimeric mice can be generated.

In 2007, each of the two research groups Yamanaka and Junying YU successfully reprogrammed human somatic cells into iPS cells respectively, wherein, the former transduced Oct3/4, Sox2, c-Myc and Klf4 into human epidermal fibroblasts by using a retrovirus, while the latter introduced Oct3/4, Sox2, Nanog and Lin28 into foreskin cells by using a lentivirus. Both of the analysis on gene expression profiling and the analysis on the methylation of the promoter regions of genes Oct3/4 and Nanog showed that human iPS cell lines are extremely resemble to the corresponding embryonic stem cell line and all these cells can develop into tissues characteristic of three germ layers when these cells are injected into the body of a nude mouse. Furthermore, somatic cells can be successfully induced into iPSCs in rat, swine and monkey, in addition to mouse and human.

CN102816796A describes a method of inducing pluripotent stem cells using cJunDN, an N-terminal truncation of c-Jun, in combination with (a) Klf4, Sox2, and Oct4, (b) Klf4, Sox2, and c-Myc, (c) Klf4, Oct4, and c-Myc, or (d) KLf4 and Sox2. EP2647700 describes a method for increasing the efficiency of inducing pluripotent stem cells using Jhdmlb in combination with (a) Oct4, (b) Oct4, Klf4, and Sox2, (c) Oct4, Klf4, c-Myc, and Sox2. US2010/190250 describes a method for rejuvenating somatic cells using Oct4, Sox2, Nanog, the ID family member proteins (e.g. Id1), and/or the BcI6 family member proteins. Chiou et al. (2013) Control of Oxidative Stress and Generation of Induced Pluripotent Stem Cell-like Cells by Jun Dimerization Protein 2, Cancers, 5:959-974, describes reprogramming of medulloblastoma cells by JDP2 and Oct4 to become iPSC-like cells.

By far, many research institutes have attempted reprogramming techniques on various kinds of cells and earned success. Cells that can be reprogrammed successfully are not limited to fibroblasts and also include hepatic cells, gastric cells, hematopoietic cells, meningeal cells, peripheral blood CD34 positive cells, keratinocytes, and so on. Besides commonly used method for introducing exogenous genes into cells by using a retrovirus or lentivirus, methods for inducing reprogramming may also use other different vectors such as non-integrating adenovirus vectors, transfer elements and the like to induce reprogramming.

To sum up, the formation of induced pluripotent stem cells has great importance to regenerative medicine and life science research. Induced pluripotent stem cells are helpful for studying mammalian development mechanism, and for providing various kinds of human somatic cells by in vitro differentiation to conduct medicine research (in particular research on selection of chemotherapeutic drugs). Also, induced pluripotent stem cells are expected to be used in clinic transplantation, stem cell therapy, etc. In view of these application prospects of induced pluripotent stem cells, it is now desirable to find other combined factors for replacing commonly used transcription factor combinations to increase the reprogramming efficiency and reduce the cell mutation during reprogramming.

### SUMMARY OF THE INVENTION

The present disclosure provides a method for preparing an induced pluripotent stem cell, comprising introducing a composition for promoting the formation of an induced pluripotent stem cell into a somatic cell, wherein, said composition comprises: (i) a c-Jun antagonist, and one group of factors selected from the following seven groups of factors: (1) Sox2, Klf4 and c-Myc, (2) Klf4 and c-Myc, (3) Oct3/4, Klf4 and c-Myc, (4) Sox2, Nanog and Lin28, (5) Oct3/4, Nanog and Lin28, (6) Oct3/4, Klf and Sox2, and (7) Klf4 and Sox2; or, said composition comprises: (ii) a c-Jun antagonist, Jhdmlb and Id1; and at least one of Glis1, Sall4 and Lrhl; or, said composition comprises: (iii) a c-Jun antagonist, Jhdmlb and Id1; and at least one of Oct4, Klf4, Sox2, Lin28, Esrrb, Lefl, Utfl and miRNA C, and, wherein, when the c-Jun antagonist is c-JunDN, said composition does not comprise the following four groups of factors: (1) Sox2, Klf4 and c-Myc, (2) Oct3/4, Klf4 and c-Myc, (3) Oct3/4, Klf and Sox2, or (4) Klf4 and Sox2.

The present disclosure also provides a composition for promoting the formation of an induced pluripotent stem cell, wherein, said composition comprises: (i) a c-Jun antagonist, and one group of factors selected from the following seven groups of factors: (1) Sox2, Klf4 and c-Myc, (2) Klf4 and c-Myc, (3) Oct3/4, Klf4 and c-Myc, (4) Sox2, Nanog and Lin28, (5) Oct3/4, Nanog and Lin28, (6) Oct3/4, Klf and Sox2, and (7) Klf4 and Sox2; or, said composition comprises: (ii) a c-Jun antagonist, Jhdmlb and Id1; and at least one of Glis1, Sall4 and Lrhl; or, said composition comprises: (iii) a c-Jun antagonist, Jhdmlb and Id1; and at least one of Oct4, Klf4, Sox2, Lin28, Esrrb, Lefl, Utfl and miRNA C, and, wherein, when the c-Jun antagonist is c-JunDN, said composition does not comprise the following four groups of factors: (1) Sox2, Klf4 and c-Myc, (2) Oct3/4, Klf4 and c-Myc, (3) Oct3/4, Klf and Sox2, or (4) Klf4 and Sox2.

In some examples of the present disclosure, the composition for promoting the formation of an induced pluripotent stem cell comprises: (a) a c-Jun antagonist, and (b) Sox2, Klf4 and c-Myc; or, the composition comprises: (a) a c-Jun antagonist, and (b) Oct3/4, Klf4 and c-Myc; or, the composition comprises: (a) a c-Jun antagonist, and (b) Sox2 and Klf4; or, the composition comprises: (a) a c-Jun antagonist, and (b) Oct3/4, Klf and Sox2; or, the composition comprises: (a) a c-Jun antagonist, Jhdmlb and Id1; and (b) Glis1, Sall4 and Lrhl; or, the composition comprises: (a) a c-Jun antagonist, Jhdmlb and Id1, and (b) Oct4; or, the composition comprises: (a) a c-Jun antagonist, Jhdmlb and Id1; and (b) Klf4; or, the composition comprises: (a) a c-Jun antagonist, Jhdmlb and Id1; and (b) Sox2; or, the composition comprises: (a) a c-Jun antagonist, Jhdmlb and Id1; and (b) Lin28; or, the composition comprises: (a) a c-Jun antagonist, Jhdmlb and Id1; and (b) Esrrb; or, the composition comprises: (a) a c-Jun antagonist, Jhdmlb and Id1; and (b) Lefl; or, the composition comprises: (a) a c-Jun antagonist, Jhdmlb and Id1; and (b) Utfl; or, the composition comprises: (a) a c-Jun antagonist, Jhdmlb and Id1; and (b) miRNA C.

In one aspect, the present invention provides a method for preparing an induced pluripotent stem cell, comprising introducing a composition for promoting the formation of an induced pluripotent stem cell into a mammalian somatic cell, wherein, said composition comprises nucleic acids encoding the following polypeptides: a c-Jun antagonist, Jhdm1b, Id1, and at least one factor selected only from Glis1, Sall4 and Lrh1; wherein the c-Jun antagonist comprises:
c-JunDN having the amino acid sequence of SEQ ID NO. 2 or a functional variant thereof, wherein the functional variant thereof has a bZIP domain but lacks a transactivation domain and shows at least 70% homology to SEQ ID NO. 2, or
Jdp2 having the amino acid sequence of SEQ ID NO. 3 or a functional variant thereof, wherein the functional variant thereof has a bZIP domain but lacks a transactivation domain and shows at least 70% homology to SEQ ID NO. 3; and
wherein, when the c-Jun antagonist is c-JunDN, said composition does not comprise nucleic acids encoding the following four groups of polypeptides: (1) Sox2, Klf4 and c-Myc, (2) Oct3/4, Klf4 and c-Myc, (3) Oct3/4, Lkf and Sox2, or (4) Klf4 and Sox2.

The invention also provides a composition for promoting the formation of an induced pluripotent stem cell, wherein, said composition comprises nucleic acids encoding the following polypeptides: a c-Jun antagonist, Jhdm1b, Id1, and at least one factor selected only from Glis1, Sall4 and Lrhl; wherein the c-Jun antagonist comprises:
c-JunDN having the amino acid sequence of SEQ ID NO. 2 or a functional variant thereof, wherein the functional variant thereof has a bZIP domain but lacks a transactivation domain and shows at least 70% homology to SEQ ID NO. 2, or
Jdp2 having the amino acid sequence of SEQ ID NO. 3 or a functional variant thereof, wherein the functional variant thereof has a bZIP domain but lacks a transactivation domain and shows at least 70% homology to SEQ ID NO. 3, and
wherein, when the c-Jun antagonist is c-JunDN, said composition does not comprise nucleic acids encoding the following four groups of polypeptides: (1) Sox2, Klf4 and c-Myc, (2) Oct3/4, Klf4 and c-Myc, (3) Oct3/4, Lkf and Sox2, or (4) Klf4 and Sox2.

Further aspects and embodiments of the present invention are recited in the appended claims.

c-Jun antagonists include a c-Jun antagonistic factor having a bZIP domain but lacking a transactivation domain and a compound, a nucleic acid, a protein and RNA antagonizing c-Jun activity. c-Jun antagonists may comprise a truncated c-Jun gene (c-JunDN) or Jdp2 or a functional variant thereof. The functional variant includes: a functional variant that still has a bZIP domain but lacks a transactivation domain, in which 1 to 100 amino acids among c-JunDN amino acids are substituted, inserted and/or deleted, wherein, c-JunDN has an amino acid sequence of SEQ ID NO. 2; Jdp2 has an amino acid sequence of SEQ ID NO. 3; the functional variant of c-JunDN has an amino acid sequence showing at least 70% homology to SEQ ID NO. 2; and the functional variant of Jdp2 has an amino acid sequence showing at least 70% homology to SEQ ID NO. 3.

In some embodiments of the method of the present invention, the somatic cells are selected from mouse somatic cells and human somatic cells. Preferably, the mouse somatic cells and the human somatic cells are selected from the group consisting of fibroblasts, bone marrow derived mononuclear cells, skeletal muscle cells, fat cells, peripheral blood mononuclear cells, macrophages, neural stem cells, hepatic cells, keratinocytes, oral keratinocytes, hair follicle dermal cells, gastric epithelial cells, lung epithelial cells, synovial cells, renal cells, skin epithelial cells and osteoblast cells.

In yet another aspect, the present invention provides an induced pluripotent stem cell obtained according to the above method for preparing an induced pluripotent stem cell.

In still another aspect, the present invention provides uses of the composition for promoting the formation of an induced pluripotent stem cell in the preparation of an induced pluripotent stem cell.

### BRIEF DESCRIPTION OF THE DRAWINGS

FIG. 1A shows expression levels of c-Jun in mouse embryonic stem cells, induced pluripotent stem cells and mouse embryonic fibroblasts, compared with Oct4 and Nanog.
FIG. 1B shows expression levels of c-Jun in mouse embryonic stem cells, induced pluripotent stem cells and mouse embryonic fibroblasts, compared with Oct4.
FIG. 1C shows protein expression levels of c-Jun in c-Jun^{+/+}, c-Jun^{-/-} and c-Jun^{+/-} mouse embryonic stem cells.
FIGS. ID and IE show changes in cell number of c-Jun^{+/+}, c-Jun^{-/-}, c-Jun^{+/-}mouse embryonic stem cells and mouse embryonic fibroblasts with the increase of cell culture days, respectively.
FIG. 1F shows expression levels of pluripotent genes in c-Jun homozygous knockout mouse embryonic stem cells.
FIG. 1G shows that c-Jun^{-/-} mouse embryonic stem cells can differentiate into cells having three germ layers in EB ball experiment.
FIG. 1H schematically shows the course of establishing embryonic stem cells for DOX inductively expressing c-Jun.
FIG. 2A shows clone number of the stem cells growing in a serum-containing medium and a serum-free medium in c-Jun+OKS system and c-Jun+OKSM system.
FIG. 2B shows micrographs of induced pluripotent stem cells obtained in c-Jun+OKS system, control +OKS system, c-Jun+OKSM system, and OKSM+control system, wherein the control is OKS or OKSM system without the addition of c-Jun and the scale bar is 250µm.
FIG. 2C shows expression levels of c-Jun and clone number of GFP⁺ iPS under different DOX concentration conditions.
FIG. 2D shows the changes of GFP⁺ iPS clone number with c-Jun expression with the increase of days using DOX.
FIG. 3A is a schematic diagram showing the structures of wild-type c-Jun (c-JunWT), Ser63 site phosphorylation mutant (c-Jun S63A), Ser73 site phosphorylation mutant (c-Jun S73A), Ser63 and Ser73 site phosphorylation mutant (c-Jun S63A/S73A), and truncated c-Jun (c-JunDN) located between amino acids 170-334 and having a bZIP domain but lacking a transactivation domain.
FIG. 3B shows the changes of GFP⁺ iPS clone number after adding c-JunWT, c-Jun S63A, c-Jun S73A, c-Jun S63A/S73A and c-JunDN to OKS system and OKSM system, respectively, wherein the controls are OKS and OKSM systems themselves .
FIG. 3C shows micrographs of the obtained induced pluripotent stem cells, wherein the controls are OKS and OKSM systems themselves without the addition of any other factor and the scale bar is 250µm.
FIG. 4A is a schematic diagram showing protein sequences of c-JunWT, c-Jun-bZIP, a.a 75-334, c-JunDN, a.a 254-334, a.a 274-334 and a.a 170-282.
FIG. 4B shows the proportion of green fluorescent cells obtained by analysis using a flow cytometer after introducing the above-mentioned c-Jun factor mutants into OKS system.
FIG. 4C shows the GFP⁺ iPS clone number obtained in iSF1 culture medium or mES culture medium supplemented with vitamin C, in OKS system with the addition of c-JunDN and c-Jun-bZIP respectively, wherein the control is OKS system itself and the vector is pMXs retroviral vector.
FIG. 5A is a schematic diagram showing protein sequences of wild-type c-Jun (c-JunWT), c-JunDN and Jdp2.
FIG. 5B shows clone number of GFP⁺ iPS obtained after adding c-JunWT, c-JunDN and Jdp2 to OKS system, wherein the control is OKS system itself.
FIG. 5C is a schematic diagram showing protein sequences of different kinds of Jdp2 mutants.
FIG. 5D shows the proportion of green fluorescent cells obtained by analysis using a flow cytometer after introducing the above-mentioned Jdp2 mutants into OKS system.
FIG. 6A shows exogenous gene integration maps of induced pluripotent stem cells (iPSCs) obtained by PCR analysis of mouse embryonic fibroblasts in c-JunDN/Jdp2+KSM system and c-JunDN/Jdp2+KS system.
FIG. 6B shows immune fluorescence images of induced pluripotent stem cells cultured in c-JunDN/Jdp2+KSM system and c-JunDN/Jdp2+KS system.
FIG. 6C shows analysis results of methylation status of CpG islands located in Oct4 and Nanog promoter regions.
FIG. 6D shows the karyotype of c-JunDN/Jdp2+KSM and c-JunDN/Jdp2+KS induced pluripotent stem cells cultured.
FIG. 6E shows chimeric mice obtained by injecting induced pluripotent stem cells generated in c-JunDN/Jdp2+KSM system and c-JunDN/Jdp2+KS system.
FIG. 7A shows RNA-seq data analysis results of mouse embryonic fibroblasts infected with the control, c-JunFL, c-JunDN and Jdp2.
FIG. 7B shows the genes up-regulated and down-regulated by c-Jun in mouse embryonic fibroblasts. c-Jun activates cell proliferation relative genes and down-regulates cell adhesion genes.
FIG. 7C shows influences of c-Jun, Oct4, c-JunDN and Jdp2 on cell number of mouse embryonic fibroblasts with the increase of culture days, wherein the control is the growth of mouse embryonic fibroblasts without the addtion of c-Jun, Oct4, c-JunDN and Jdp2.
FIG. 8A shows RNA-seq data analysis results of mouse embryonic fibroblasts reprogrammed in KSM, KSM+Oct4, KSM+c-JunDN and KSM+Jdp2 systems.
FIG. 8B shows the number of the genes regulated by c-JunDN, Jdp2 and Oct4, wherein they co-regulate 1100 genes.
FIG. 8C shows the up-regulated and down-regulated genes commonly regulated by c-JunDN/Oct4. c-JunDN/Oct4 co-activate stem cell maintenance relative genes and down-regulate cell differentiation relative genes.
FIG. 8D shows that representative genes Tdh, Nodal, Lefhy2, Pax1, Sqrd1 and Stx11 of mouse embryonic fibroblasts during reprogramming are co-regulated by c-JunDN, Jdp2 and Oct4, as evidenced by qRT-qPCR method.
FIG. 9A shows the GFP⁺ iPS clone number in KSM+c-JunDN and OKM+c-JunDN systems, wherein "-" indicates no c-JunDN is added and "+" indicates c-JunDN is added.
FIG. 9B shows fluorescence micrographs of induced pluripotent stem cells generated in KSM+c-JunDN system, wherein the scale bar is 250µm.
FIG. 10A shows clone number of GFP⁺ iPS obtained in KSM+Jdp2 and KS+Jdp2 systems, wherein the control is KSM or KS system without the addition of Jdp2.
FIG. 10B shows fluorescence micrographs of induced pluripotent stem cells generated in KSM+Jdp2 and KS+Jdp2 systems, wherein the scale bar is 250µm.
FIG. 11 shows clone number of GFP⁺ iPS of mouse embryonic fibroblasts generated in a six-factor (6F) system comprised of c-JunDN/Jdp2, Jhdm1b, Id1/3, Glis1, Sall4 and Lrhl and clone number of GFP⁺ iPS of mouse embryonic fibroblasts generated in a five-factor system with any one factor eliminated from the six factors.
FIG. 12 shows fluorescence micrographs of induced pluripotent stem cells generated in the 6F system, wherein the scale bar is 250µm.
FIG. 13A shows PCR analysis results of pluripotent stem cells generated in the 6F system.
FIG. 13B shows the karyotype of 6F induced pluripotent stem cells.
FIG. 13C shows a photograph of the chimeric mouse obtained by injecting 6F induced iPSCs into the blastula of a donor mouse and then transplanting the injected blastocoele into the uterus of a pseudo-pregnant female mouse.

### DETAILED DESCRIPTION OF PREFERRED EMBODIMENTS

Unless otherwise defined, all technical and scientific terms used herein have the same meaning as commonly understood by one of ordinary skill in the art to which this invention belongs.

### 1. Method for Preparing iPSCs

### Overview

In general, the present invention provides a method for preparing an induced pluripotent stem cell (iPSC) using a composition comprising a c-Jun antagonist and a composition comprising a c-Jun antagonist for promoting the formation of iPSCs as used in the method, wherein, said composition comprises a c-Jun antagonist and one or more kinds of other factors as mentioned herein, and the c-Jun antagonist is for example a c-Jun antagonistic factor having a bZIP domain but lacking a transactivation domain, and a compound, an antibody or a fragment of an antibody that antagonizes c-Jun activity.

In one aspect, the present invention provides a method for preparing an induced pluripotent stem cell (iPSC), comprising introducing a composition for promoting the formation of an induced pluripotent stem cell (iPSC) into a somatic cell, wherein, said composition comprises:
(i) a c-Jun antagonist, and one group of factors selected from the following seven groups of factors: (1) Sox2, Klf4 and c-Myc, (2) Klf4 and c-Myc, (3) Oct3/4, Klf4 and c-Myc, (4) Sox2, Nanog and Lin28, (5) Oct3/4, Nanog and Lin28, (6) Oct3/4, Klf and Sox2, and (7) Klf4 and Sox2; or
said composition comprises:
(ii) a c-Jun antagonist, Jhdmlb and ld1; and at least one of Glis1, Sall4 and Lrhl; or,
said composition comprises:
(iii) a c-Jun antagonist, Jhdmlb and ld1; and at least one of Oct4, Klf4, Sox2, Lin28, Esrrb, Lefl, Utfl and miRNA C.

### c-Jun Antagonist

c-Jun is a proto-oncogene known to play an important role in cell proliferation, survival, and transformation. It was the first carcinogenic factor found to have DNA sequence specific binding capability. c-Jun regulates the expression of various genes by binding to conservative sequences TGA(C/G)TCA located in promoter or enhancer regions. Once anchored on the chromatin, c-Jun interacts with the general transcription factor complex through its transactivation domain located at the N-terminus to activate the expression of target genes. c-Jun can also dimerize through its leucine zipper domain with other factors of the AP-1 family to generate diversity of transcription regulatory complexes. As such, c-Jun has been shown to regulate a plethora of downstream targets implicated in carcinogenesis, cell proliferation, apoptosis, etc.

"c-Jun antagonist" as described herein refers to a molecule capable of blocking or reducing the activity of c-Jun, for example, a small molecular compound, a substance derived from nucleic acid and the like that can bind to a natural binding partner (such as AP-1) of c-Jun but does not activate a downstream gene of c-Jun and can enter a cell. In some embodiments, the c-Jun antagonist comprises a small molecular compound capable of binding c-Jun or AP-1, e.g., a mRNA drug that can reduce the expression of c-Jun, for example, FSK (Forskolin, Mao Housu, a commonly used adenosine cyclase activator), HDAC (histone deacetylase) inhibitor, etc. In some embodiments, the c-Jun antagonist comprises a nucleic acid capable of binding to c-Jun or AP-1, for example, aptamer. In some embodiments, the c-Jun antagonist comprises a protein capable of inhibiting the expression of c-Jun, for example, antisense nucleic acid (e.g. DNA or RNA or a hybrid thereof).

In some embodiments, the c-Jun antagonist includes a variant of c-Jun, for example, a functional variant of c-Jun or a fragment of c-Jun. The term "functional variant" as used herein refers to inclusion of for example only conservative changes or changes in non-critical residues or in non-critical regions and preservation of functions of an original polypeptide. Functional variant may also comprise replacement of similar amino acids, which leads to unchanged or insignificantly changed functions. Said replacement of amino acids includes replacement in amino acid sequences by substituting, eliminating, inserting, fusing, truncating one or more amino acids (for example 1-100 amino acids, e.g., 1-90 amino acids, e.g., 1-80 amino acids, e.g., 1-70 amino acids, e.g., 1-60 amino acids, e.g., 1-50 amino acids, e.g., 1-40 amino acids, e.g., 1-30 amino acids) or any combination thereof. In some embodiments, a plurality of amino acids (e.g., 50 amino acids, e.g., 40 amino acids) are inserted into a truncated amino acid to enhance the stability and expression efficiency of the functional variant of the c-Jun antagonist.

In the present invention, various different molecules can be used as c-Jun antagonists. In some embodiments, the following steps can be used to select a new c-Jun antagonist (T.S.Huang, et al., Proc. Natl. Acad. Sci. USA, 88, 5292, 1991, B. L. Bennett et al., Proc. Natl. Acad. Sci. USA, 98, 13681, 2001).
(1) Reporter Gene Assay: a candidate antagonist and a reporter gene plasmid are imported into a cell together by using AP-1 reporter gene luciferase plasmid, and fluorescence intensity is detected with a fluorescence illuminance meter after 36 hours.
(2) EMSA (gel migration test, Electrophoretic Mobility Shift Assay): a cell is lysed upon treated with a candidate antagonist, followed by co-incubation with a probe comprising AP-1 binding site. Then, electrophoretic separation is conducted using 4% non-denatured polyacrylamide gel, followed by drying and developing the polyacrylamide gel. It is examined whether the antagonist has any effect on the binding of c-Jun to the probe comprising AP-1 binding site.
(3) Western Blotting Assay: a cell is lysed upon treated with a candidate antagonist and the phosphorylation level of c-Jun in the cell is detected using a phosphorylated c-Jun antibody.
(4) RT-PCR detection: A cell is treated with a candidate antagonist. mRNA is extracted from the cell and the extracted mRNA is reversely transcripted to obtain cDNA. Then fluorescence quantitative PCR analysis is conducted using a specific c-Jun primer to detect mRNA expression levels of c-Jun in the sample.

### Preparation of iPSCs

In one aspect, the present invention provides a method for preparing iPSCs by using a c-Jun antagonist and one group of factors selected from the following seven groups of factors:
(1) Sox2, Klf4 and c-Myc,
(2) Klf4 and c-Myc,
(3) Oct3/4, Klf4 and c-Myc,
(4) Sox2, Nanog and Lin28,
(5) Oct3/4, Nanog and Lin28,
(6) Oct3/4, Klf and Sox2, and
(7) Klf4 and Sox2.

In some embodiments of the present invention, the present invention utilizes a composition comprising a c-Jun antagonist, and Sox2, Klf4 and c-Myc to prepare iPSCs, or, the present invention utilizes a composition comprising a c-Jun antagonist, and Oct3/4, Klf4 and c-Myc to prepare iPSCs, or, the present invention utilizes a composition comprising a c-Jun antagonist, and Oct3/4, Klf4 and c-Myc to prepare iPSCs, or, the present invention utilizes a composition comprising a c-Jun antagonist, and Sox2 and Klf4 to prepare iPSCs.

In another aspect, the present invention provides a method for preparing iPSCs by using a non-Yamanaka factor, i.e., c-Jun antagonist, Jhdmlb and ld1, and at least one of Glis1, Sall4 and Lrhl.

In a preferred embodiment of the present invention, the present invention utilizes a c-Jun antagonist, Jhdmlb and Id1; and Glis1, Sall4 or Lrhl to prepare iPSCs.

In still another aspect, the present invention provides a method for preparing iPSCs by using a c-Jun antagonist, Jhdmlb and Id1, and at least one factor selected from the group consisting of Oct4, Klf4, Sox2, Lin28, Esrrb, Lefl, Utfl and miRNA C.

In some embodiments of the present invention, the present invention utilizes a c-Jun antagonist, Jhdmlb and ld1 as well as Oct4 to prepare iPSCs, or, the present invention utilizes a c-Jun antagonist, Jhdmlb and ld1 as well as Klf4 to prepare iPSCs, or, the present invention utilizes a c-Jun antagonist, Jhdmlb and ld1 as well as Sox2 to prepare iPSCs, or, the present invention utilizes a c-Jun antagonist, Jhdmlb and ld1 as well as Lin28 to prepare iPSCs, or, the present invention utilizes a c-Jun antagonist, Jhdmlb and ld1 as well as Esrrb to prepare iPSCs, or, the present invention utilizes a c-Jun antagonist, Jhdmlb and ld1 as well as Lefl to prepare iPSCs, or, the present invention utilizes a c-Jun antagonist, Jhdmlb and ld1 as well as Utfl to prepare iPSCs, or, the present invention utilizes a c-Jun antagonist, Jhdmlb and ld1 as well as miRNA C to prepare iPSCs.

Generally speaking, the method for preparing iPSCs includes the process of inducing a mammalian somatic cell into a pluripotent stem cell. Said method is accomplished by expressing an exogenous or endogenous polypeptide (especially a protein that plays an important role in maintaining or regulating embryonic stem cell self-renewal and/or pluripotency). The protein that plays an important role in maintaining or regulating embryonic stem cell self-renewal and/or pluripotency is for example transcription factors Oct3/4, Sox2, Klf4 and c-Myc highly expressed in embryonic stem cells. Expressing these transcription factors may include introducing an expression vector that encodes a target polypeptide into a cell, transducing a cell by using a recombinant virus, introducing an exogenous purified target polypeptide into a cell, contacting a cell with a non-naturally occurring inducing reagent that induces the expression of an endogenous gene (for example, Oct3/4, Sox2, Klf4 and c-Myc) encoding a target polypeptide, contacting a cell with one or more inducing reagents or inducing factors, or, contacting a cell with other biological, chemical or physical reagents that induce the expression of a target polypeptide encoding gene (for example, endogenous genes Oct3/4, Sox2, Klf4 and c-Myc). For example, in some cases (depending on the type of the cell to be induced), one or more exogenous inducing factors can be used in combination with a chemical inducing reagent, which is for example histone deacetylase (for instance, valproic acid), histone methyltransferase inhibitor (e.g., BIX01294), DNA demethylation reagent (for example, 5-cytidine), L-type calcium channel agonist (for example, BayK8644), or TFG-beta receptor inhibitor. The basic steps for inducing cell reprogramming comprise: (1) collecting cells from a donor, (2) inducing the cells by for example enabling a polypeptide e.g. Oct3/4, Sox2, Klf4 and c-Myc to express, (3) selecting pluripotent stem cells, (4) isolating and cloning, and (5) optionally, storing the cells. Each step of the above-mentioned steps for inducting cell reprogramming includes cell culture and amplification. The finally obtained induced pluripotent stem cells can be used in various application areas, such as cell transplantation therapy, and so on.

As used in the method for inducing cell reprogramming of the present invention, the cell from a donor may be various kinds of mammalian cells. Examples of suitable mammalian cell population include but are not limited to: fibroblasts, bone marrow derived mononuclear cells, skeletal muscle cells, fat cells, peripheral blood mononuclear cells, macrophage, neural stem cells, hepatic cells, keratinocytes, oral keratinocytes, hair follicle dermal cells, gastric epithelial cells, lung epithelial cells, synovial cells, renal cells, skin epithelial cells and osteoblast cells.

Donor-derived cells may also be originated from different types of tissues, such as, bone marrow, skin (e.g. dermis, epidermis), scalp tissue, muscle, adipose tissue, peripheral blood, skeletal muscle or smooth muscle. The cells may also be derived from neonatal tissues, including but not limited to: umbilical cord tissue (e.g., umbilical cord, cord blood, umbilical cord blood vessel), amnion, placenta and other different neonatal tissues (e.g., bone marrow fluid, muscle, adipose tissue, peripheral blood, skin, skeletal muscle, and so on).

Donor-derived cells preferably are human cells, but may also be derived from cells of non-human mammals. Non-human mammals include but are not limited to: non-human primates (such as, apes, monkeys, chimpanzees), rodent animals (such as, mice, rats), cattle, pigs, sheep, horses, dogs, cats and rabbits.

In some embodiments of the present invention, cells can be collected from patients suffered from various kinds of diseases, or cells can be collected from non-sick humans. Under some circumstances, cells are collected from humans suffered from or at risk of suffering from the following diseases, for example chronic disease (such as cardiovascular disease), eye disease (such as macular degeneration), hearing disorder (such as deaf), diabetes, cognitive dysfunction, schizophrenia, depression, manic depressive disorders , dementia, neurodegenerative disease, Alzheimer disease, Parkinson's disease, multiple sclerosis, osteoporosis, hepatopathy, nephropathy, autoimmune disease, asthma or proliferative disorder (such as cancer). Under some circumstances, cells may be collected from humans suffered from or at risk of suffering from the following diseases, for example acute diseases, such as, apoplexy, spinal cord injury, burn, trauma, and so on.

In some embodiments, as used in the method for preparing iPSCs of the present invention, the amino acid sequence of other induced factors (IF) such as Oct3/4, Klf4, Sox2, c-Myc, Nanog and Lin28 may be naturally occurring amino acid sequence of these factors, for example, human or mouse Oct3/4, human or mouse Klf4, human or mouse Sox2, human or mouse c-Myc, human or mouse Nanog and human or mouse Sox2, or may be a variant of naturally occurring amino acid sequence of IFs (i.e., non-naturally occurring amino acid sequence) which however is homologous to naturally occurring amino acid sequence in terms of function and structure.

A method for evaluating the homology of two or more amino acid sequences in terms of function or structure usually includes determining the percent consistency between amino acid sequences. "Percent consistency" refers to the number of identical residues (i.e., amino acids or nucleotides) at positions common to the compared sequences. Sequence alignment and comparison may be carried out by the standard algorithms of the art (for example, Smith and Waterman, 1981, Adv. Appl. Math. 2:482; Needleman and Wunsch, 1970, J. MoI. Biol. 48:443; Pearson and Lipman, 1988, Proc. Natl. Acad. Sci., USA, 85:2444) or a computerized version of these algorithms (Wisconsin Genetics Software Package Release 7.0, Genetics Computer Group, 575 Science Drive, Madison, WI), wherein said computerized version is publicly available as BLAST and FASTA. Additionally, the ENTREZ available from the National Institutes of Health (Bethesda MD) may be used for sequence comparison. When BLAST and gapped BLAST programs are used, the default parameters of the respective programs (such as BLASTN, which is available on the internet site of the National Center for Biotechnology Information) may be used. In one embodiment, GCG with a gap weight of 1 may be used to determine the percent identity between two sequences, such that each amino acid gap is given a weight as if it is a single amino acid mismatch between the two sequences. Alternatively, ALIGN program (version 2.0), which is a part of GCG (Accelrys, San Diego, CA) sequence alignment software package, may be used.

"Oct3/4" used herein indicates a member of the family of Octamer (Oct) transcription factors and plays a crucial role in maintaining pluripotency. The absence of Oct3/4 in Oct3/4+cells (such as blastomere and embryonic stem cells) leads to spontaneous trophoblast differentiation and thus the presence of Oct3/4 gives rise to the pluripotency and differentiating potential of embryonic stem cells. Various other genes in the Oct family, including Oct3/4's close relatives, Oct1 and Oct6, fail to induce pluripotency, thus demonstrating the exclusiveness of Oct3/4 to the process of pluripotency induction.

The Sox family of genes is associated with the maintenance of pluripotency similar to Oct3/4, although it is associated with multipotent stem cells and unipotent stem cells in contrast with Oct3/4, which is exclusively expressed in pluripotent stem cells. While Sox2 was the initial gene used for induction by Yamanaka et al., Other genes in the Sox family have been found to work as well in the induction process. Sox1 yields iPSCs with a similar efficiency as Sox2, and Sox3, Sox15 and Sox18 also generate iPSCs, although with decreased efficiency.

In the preparation of induced pluripotent stem cells, at least one Oct member (such as Oct3/4) and at least one Sox member (such as Sox2) are necessary for promoting the generation of pluripotency. Yamanaka et al. reported that Nanog may not be necessary for the generation of induced pluripotency (Kazutoshi Takahashi, et al., Cell, 131:1-12, 2007), although Junying Yu et al. reported that Nanog may be used as one of the factors for promoting the formation of iPSCs and Nanog certainly enhances reprogramming efficiency in a dose-dependent manner (Junying Yu, et al. Science, 318: 1917-1920, 2007).

Lin28 is an mRNA binding protein expressed in embryonic stem cells and embryonic carcinoma cells associated with differentiation and proliferation. Junying Yu et al. demonstrated it is a factor in iPSC generation, although it is unnecessary (Junying Yu, et al. Science, 318: 1917-1920, 2007).

Klf4 of the Klf family of genes was initially identified by Yamanaka et al., and confirmed by Jaenisch et al. as a factor useful for the generation of mouse iPSCs (Jaenisch, Science, 240:1468-1474, 1998), and was demonstrated by Yamanaka et al. as a factor useful for the generation of human iPSCs (Kazutoshi Takahashi, et al., Cell, 131:1-12, 2007). Klf2 and Klf4 are factors capable of generating iPSCs, and related genes Klf1 and Klf5 do as well, although with reduced efficiency.

The Myc family of genes are proto-oncogenes implicated in cancer. It was demonstrated by Yamanaka et al. and Jaenisch et al. that c-Myc is a factor implicated in the generation of mouse iPSCs (Jaenisch, Science, 240 : 1468-1474, 1998). Yamanaka et al. confirmed that c-Myc is a factor implicated in the generation of human iPSCs. However, c-Myc is unnecessary for the generation of human iPSCs. n-Myc and L-Myc have been identified to induce the generation of iPSCs with an efficiency similar to c-Myc.

Jhdmlb is a member of the family of JmjC-domain-containing histone demethylase (JHDM) that is evolutionarily conserved and widely expressed. It is also called Fbx110.

Glis1 is a Gli-like transcription factor 1 (Glis family zinc finger 1), which is enriched in unfertilized oocytes and in embryos at the single cell stage. Glis1 can promote multiple early steps of reprogramming and promote the formation of induced pluripotent stem cells in place of c-Myc.

Sall4 is a sal-like gene 4 (sal-like4, SALL4) located at chromosome 20q13.13-13.2. Its encoding protein is a transcription factor containing eight zinc finger motifs. Sall4 gene plays a key role in early embryonic development, organ formation as well as embryonic stem cell proliferation and pluripotency maintenance.

Lrh1, a member of the subfamily of nuclear receptors FTZ-F1, can replace Oct4.

Id1, a downstream factor of bone morphogenetic protein (BMP), can promote the reprogramming of somatic cells in Oct4 and Sox2 2-factor system.

Esrrb is a protein of the family of orphan receptors, which plays an important role in embryonic stem cells. Specifically, silent Esrrb may lead to differentiation of embryonic stem cells in the presence of LIF (leukocyte inhibitory factor). In addition, proteomics research finds that Esrrb may have protein-protein interaction with Nanog.

Lefl, lymphoid enhancer-binding factor 1, is a nuclear protein having a molecular weight of 48kD and expressed in B and T precusor cells. Lefl binds to functionally important sites in T-cell receptor α(TCRA) enhancer and presents highest enhancer activity. Lefl belongs to regulatory protein family homologous to high mobility group protein-1 (HMG1).

Utfl, undifferentiated embryonic cell transcription factor 1, is highly expressed in stem cells and located in nucleus. Utfl may adjust chromatin state of stem cells and inhibit differentiation of stem cells.

miRNA C represents miRNA gene cluster 302-367. miRNA gene cluster improves the reprogramming efficiency of somatic cells by accelerating the conversion of mesenchymal cells to epithelial cells (B. Liao et al., J Biol Chem, 286 (19) : 17359-17364, 2011).

A variety of pluripotent factors have been known to a person skilled in the art. Preferably, the pluripotent factors include Oct4, Sox2, c-Myc, Klf4, Esrrb, Nanog and Lin28. The aforementioned pluripotent factors may be derived from any source according to the cells to be introduced. Preferable pluripotent factors are mouse pluripotent factors and variants thereof, for example, Sox2, NCBI accession number: NM_011443.3; Oct4, NCBI accession number: NM_013633.2; Klf4, NCBI accession number: NM_010637.2; c-Myc, NCBI accession number: NM_010849.4; Nanog, NCBI accession number: NM_028016; Lin28, NCBI accession number: NM_145833. c-Myc may be replaced by its mutant L-myc (NCBI accession number: NM_008506.2) or N-Myc (NCBI accession number: NM_008709.3).

### Method for Introducing into Cells a Factor or Composition for Promoting the Reprogramming of Somatic Cells

iPSCs are typically prepared by transfection of certain stem cell-associated genes into non-pluripotent cells (such as fibroblasts). Transfection is typically achieved through integrating viral vectors in the current practice, such as retroviruses. Transfected genes include the master transcriptional regulators (such as Oct3/4 and Sox). After a critical period in transfection, small number of transfected cells begin to become morphologically and biochemically similar to pluripotent stem cells, and are typically isolated through morphological selection, doubling time, or through a reporter gene and antibiotic infection.

In 2007, iPSCs were inductively generated from adult somatic cells by each of the two research groups Yamanaka and Junying YU, respectively. Yamanaka's research group had successfully transformed human fibroblasts into induced pluripotent stem cells using four pivotal genes Oct3/4, Sox2, Klf4 and c-Myc with a retroviral system. Junying Yu's research group had successfully transformed human fibroblasts into induced pluripotent stem cells using Oct4, Sox2, Nanog and Lin28 with a lentivirus system.

In some embodiments, the reprogramming vector may be a viral vector, for example, a retroviral vector, to express these reprogramming factors in cells.

### Vectors

One of skill in the art would be well equipped to construct a vector through standard recombinant techniques (see, for example, Sambrook and Russell, Molecular Cloning: A Laboratory Manual 3rd Ed., Cold Spring Harbor Laboratory Press, 2001; and Ausubel et al., Current Protocols in Molecular Biology, GreenePubl. Assoc. Inc. & John Wiley & Sons, Inc., MA, 1994). Vectors include but are not limited to, plasmids, cosmids, viruses (bacteriophage, animal viruses, and plant viruses), and artificial chromosomes (e.g., YACs), wherein virus vectors include retroviral vectors (e.g. vectors derived from Moloney Murine Leukemia Virus (MoMLV), MSCV, SFFV, MPSV, SNV etc.), lentiviral vectors (e.g. vectors derived from HIV-1, HIV-2, SIV, BIV, FIV etc.), adenoviral (Ad) vectors (including replication competent, replication deficient and gutless forms thereof), adeno-associated viral (AAV) vectors, simian virus 40 (SV-40) vectors, bovine papilloma virus vectors, Epstein-Barr virus, herpes virus vectors, vaccinia virus vectors, Harvey murine sarcoma virus vectors, murine mammary tumor virus vectors, and Rous sarcoma virus vectors.

In some embodiments, the vector is a viral vector. Retroviral and lentiviral vectors have been successfully used in reprogramming somatic cells. Viral vectors can efficiently transduce cells and introduce their own DNA into a host cell. Viral vectors are a kind of expression construct that utilizes viral sequences to introduce nucleic acid into a cell. The ability of certain viruses to infect cells or enter cells via receptor-mediated endocytosis, and to integrate into host cell genome and express viral genes stably and efficiently has made them attractive candidates for the transfer of exogenous nucleic acids into cells (e.g., mammalian cells). Non-limiting examples of vectors that may be used to deliver a composition or factor combination for preparing iPSCs of the present invention are described below.

### a. Retrovirus Vectors

Retroviruses have promise as gene delivery vectors due to their ability to integrate their genes into the host genome, to transfer a large amount of foreign genetic material, to infect a broad spectrum of species and cell types and to be packaged in special cell-lines.

In order to construct a retroviral vector, a nucleic acid is inserted into the viral genome in the place of certain viral sequences to produce a virus that is replication-defective. In order to produce virions, a packaging cell line containing the gag, pol, and env genes but without the LTR and packaging components is constructed. When a recombinant plasmid containing a cDNA, together with the retroviral LTR and packaging sequences is introduced into a special cell line (e.g., by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviruses are able to infect a broad variety of cell types.

### b. Lentiviral Vectors

Lentiviruses are complex retroviruses, which, in addition to the common retroviral genes gag, pol, and env, also contain other genes with regulatory or structural function. Lentiviral vectors are well known in the art, see for example US patents No. 6,013,516 and No. 5,994,136.

Recombinant lentiviral vectors are capable of infecting non-dividing cells and can be used for both in vivo and ex vivo gene transfer and expression of nucleic acid sequences. For example, recombinant lentivirus capable of infecting a non-dividing cell wherein a suitable host cell is transfected with two or more vectors carrying the genes with packaging functions(i.e.,gag, pol and env),as well as rev and tat is described in U.S. Patent No. 5,994,136.

### c. RNA Virus Vectors

The virus vectors of the present invention can also be constructed by RNA viruses, including single-stranded RNA (ssRNA) viruses and double-stranded RNA (dsRNA) viruses. In some embodiments, the virus is a dsRNA virus, including, but not limited to: Infectious pancreatic necrosis virus, Infectious bursal disease virus, Helminthosporium victoriae virus, Cystovirus, Hypovirus, Partitivirus, Alphacryptoviruses, Betacryptoviruses, Rotavirus, and etc. In some embodiments, the virus is a ssRNA virus, including, but not limited to: Coronavirus, SARS, Okavirus, Himetobi P virus, Plautia stali intestine virus, Rhopalosiphum padi virus, Homalodisca coagulata virus 1 (HoCV-1), Acheta domesticus virus, Marnavirus, Enterovirus, Rhinovirus, Poliovirus, the common cold virus, Hepatitis A virus, Encephalomyocarditis virus, Parechovirus, Equine rhinitis B virus, Seneca Valley virus, Poliovirus, Cheravirus, Sadwavirus, Sequivirus, Torradovirus, Waikavirus, Nepovirus, Black raspberry necrosis virus, Norwalk virus, Yellow fever virus, West Nile virus, Hepatitis C virus, Dengue fever virus, Betatetravirus, Omegatetravirus, Rubella virus, Ross River virus, Sindbis virus, Chikungunya virus, Hepatitis E virus, Herpesvirus, Ebola virus, Marburg virus, Measles virus, Mumps virus, Nipah virus, Hendra virus, Rabies virus, Ippy virus, Lassa virus, Lujo virus, Lymphocytic choriomeningitis virus, Mobala virus, Mopeia virus, Amapari virus, Chapare virus, Flexal virus, Guanarito virus, Junin virus, Latino virus, Machjupo virus, Oliveros virus, Paraná virus, Pichinde virus, Pirital virus, Sabiá virus, Tacaribe virus, Tamiami virus, Whitewater Arroyo virus, Hantaan virus, Dugbe virus, Bunyamwera virus, Rift Valley fever virus, Influenzavirus, Isavirus, Thogotovirus, Hepatitis D virus, Hepatitis B virus, and etc.

### Regulating Elements

Vectors can also comprise other components or functionalities that further modulate gene delivery and/or gene expression, or that provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating cell localization); and components that influence expression of the polynucleotide.

Eukaryotic cell expression cassettes included in the vectors preferably contain (in a 5'-to-3' direction) a eukaryotic cell transcriptional promoter operatively linked to a protein-encoding sequence, splice signals (including intervening sequences), and a transcriptional termination/polyadenylation sequence .

### Promoters/Enhancers

A "promoter" is a control sequence that is a region of a nucleic acid sequence at which initiation and rate of transcription are controlled. It may contain genetic elements at which regulatory proteins and molecules may bind, such as RNA polymerase and other transcription factors, to initiate the specific transcription of a nucleic acid sequence. The phrases "operatively positioned," "operatively linked," "under the control of...," and "under the transcriptional control of... " mean that a promoter is in a correct functional location and/or orientation in relation to a nucleic acid sequence to control transcriptional initiation and/or expression of that sequence.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring an encoding sequence under the control of a promoter, one positions the 5' end of the transcription initiation site of the transcriptional reading frame downstream (i.e., 3' end) of the chosen promoter. The upstream "promoter" stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid sequence, as may be obtained by isolating the 5' non-coding sequences located upstream of the encoding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the encoding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," i.e., containing different elements of different transcriptional regulatory regions, and/or mutations that alter expression. For example, promoters that are most commonly used in recombinant DNA construction include the β-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, for example PCR. Furthermore, the control sequences that direct transcription and/or expression of sequences within non-nuclear organelles (such as mitochondria, chloroplasts, and the like), can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know promoters, enhancers, and cell type for protein expression. The promoters employed may be constitutive, tissue-specific, inducible, and/or useful under the appropriate conditions to direct high level expression of the introduced DNA segment, which is advantageous in the large-scale production of recombinant proteins and/or peptides. The promoter may be heterologous or endogenous.

Additionally any promoter/enhancer combination (as per, for example, the Eukaryotic Promoter Data Base EPDB, http://www.epd.isb-sib. Ch/) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided(either as part of the delivery complex or as an additional genetic expression construct).

Non-limiting examples of promoters include early or late viral promoters, such as, SV40 early or late promoters, cytomegalovirus (CMV) immediate early promoters, Rous sarcoma virus (RSV) early promoters; eukaryotic cell promoters, such as, e. g., beta actin promoter (Ng, Nuc. Acid Res., 17:601-615, 1989, Quitsche et al., 1989), GADPH promoter (Alexander et al., Proc. Nat. Acad. Sci. USA, 85:5092-5096, 1998; Ercolani et al., J. Biol. Chem., 263:15335-15341, 1988), metallothionein promoter (Karin et al. Cell, 36 : 371-379, 1989; Richards et al., Cell, 37 : 263-272, 1984); and concatenated response element promoters, such as cyclic AMP response element promoters (ere), serum response element promoter (sre), phorbol ester promoter (TPA) and response element promoters (tre) near a minimal TATA box. It is also possible to use human growth hormone promoter sequences (e.g., the human growth hormone minimal promoter described at Genbank, Accession No. X05244, nucleotide 283-341) or a mouse mammary tumor promoter (available from the ATCC, Cat. No. ATCC 45007). A specific example could be a phosphoglycerate kinase (PGK) promoter.

### Initiation Signals

A specific initiation signal also may be required for efficient translation of encoding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

### Multiple Cloning Sites

Vectors can include a multiple cloning site (MCS), which is a nucleic acid region that contains multiple restriction enzyme sites, any of which can be used in conjunction with standard recombinant technology to digest the vector (see, for example, Carbonelli et al., FEMS Microbiol. Lett., 177 (1): 75-82, 1999; Levenson et al., Hum. Gene Ther., 9(8):1233-1236,1998; Cocea, Biotechniques, 23(5):814-816,1997). "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Generally, a vector is linearized or fragmented using a restriction enzyme that cuts within the MCS to enable exogenous sequences to be ligated into the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### Splicing Sites

Most transcribed eukaryotic RNA molecules will undergo RNA splicing to remove introns from the primary transcripts. Vectors containing genomic eukaryotic sequences may require donor and/or receptor splicing sites to ensure proper processing of the transcript for protein expression (see, for example, Chandler et al., Proc. Natl. Acad. Sci. USA, 94 (8): 3596-601, 1997.)

### Termination Signals

The vectors or constructs of the present invention may require at least one termination signal. A "termination signal" or "terminator" is comprised of the DNA sequences involved in specific termination of an RNA transcript by an RNA polymerase. Thus, in certain embodiments a termination signal that ends the production of an RNA transcript is involved. A terminator may be necessary in vivo to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3 ' end of the transcript. RNA molecules modified with this polyA tail appear more stable and are translated more efficiently. Thus, in other embodiments involving eukaryotes, it is preferred that terminator comprises a signal for the cleavage of the RNA, and it is more preferred that the terminator signal promotes polyadenylation of the message. The terminator and/or polyadenylation site elements can serve to enhance message levels and to minimize read through from the cassette into other sequences.

Terminators contemplated for use in the present invention include any known terminator of transcription described herein or known to one of ordinary skill in the art, including but not limited to, for example, the termination sequences of genes, such as the bovine growth hormone terminator or viral termination sequences, such as the SV40 terminator. In certain embodiments, the termination signal may be a lack of transcribable or translatable sequence, such as due to a sequence truncation.

### Polyadenylation Signals

In expression, particularly eukaryotic expression, one will typically include a polyadenylation signal to effect proper polyadenylation of the transcript. Preferred embodiments include the SV40 polyadenylation signal or the bovine growth hormone polyadenylation signal. Polyadenylation may increase the stability of the transcript or may facilitate cytoplasmic transport.

### Vector Delivery

Introduction of a reprogramming vector into somatic cells with the present invention may use any suitable methods for nucleic acid delivery for transformation of a cell, as described herein or as would be known to one of ordinary skill in the art. Such methods include, but are not limited to, direct delivery of DNA ,such as by ex vivo transfection (Wilson et al., Science, 244:1344-1346, 1989; Nabel et al, 1989), by injection (U.S. Patent Nos. 5,994,624, 5,981,274, 5,945,100, 5,780,448, 5,736,524, 5,702,932, 5,656,610, 5,589,466 and 5,580,859), including microinjection (Harland and Weintraub, J. Cell Biol, 101 (3): 1094-1099, 1985; U.S. Patent No. 5,789,215); by electroporation (US Patent No. 5,384,253; Tur-Kaspa et al., Mol. Cell Biol. 6:716-718, 1986; Potter et al., Proc. Natl. Acad. Sci. USA, 81:7161-7165, 1984); by calcium phosphate precipitation (Graham and Van Der Eb, Virilogy, 52:456-467, 1973; Chen and Okayama, Mol. Cell Biol., 7 (8): 2745-2752, 1987; Rippe et al., Mol. Cell Biol. 10:689-695, 1990); by using DEAE-dextran followed by polyethylene glycol (Gopal, Mol. Cell Biol., 5:1188-1190, 1985); by direct sonic loading (Fechheimer et al., Proc Natl. Acad. Sci. USA. 84: 8463-8467, 1987); by liposome mediated transfection (Nicolau and Sene, Biochim. Biophys. Acta, 721:185-190, 1982; Fraley et al., Proc. Natl. Acad. Sci. USA, 76:3348-3352, 1979; Nicolau et al., Methods Enzymol., 149:157-176, 1987; Wong et al., Gene, 10:87-94, 1980; Kaneda et al. , Science, 243:375-378, 1989; Kato et al., J. Biol. Chem., 266:3361-3364, 1991) and receptor-mediated transfection (Wu and Wu, Biochemistry, 27:887-892, 1988; Wu and Wu, J. Biol. Chem., 262:4429-4432, 1987); by microprojectile bombardment (PCT Application Nos. WO 94/09699 and WO 95/06128; U.S. Patent Nos. 5,610,042; 5,322,783, 5,563,055, 5,550,318, 5,538,877 and 5,538,880); by agitation with silicon carbide fibers (Kaeppier et al., Plant Cell Reports, 9:415-418, 1990; U.S. Patents No. 5,302,523 and No. 5,464,765); by agrobacterium-mediated transformation (U.S. Patent Nos. 5,591,616 and 5,563,055); by PEG-mediated transformation of protoplasts (Omirulleh et al., Plant Mol. Biol., 21 (3): 415-428, 1993; U.S. Patents No. 4,684,611 and No. 4,952,500); by desiccation/inhibition-mediated DNA uptake (Potrykus et al., Mol. Gen. Genet., 199 (2): 169-177, 1985), and any combination of such methods. Through the application of the above-mentioned techniques and the like, organelle(s), cell(s), tissue(s) or organism(s) may be stably or transiently transformed.

### Liposome-Mediated Transfection

In some embodiments of the present invention, a nucleic acid may be entrapped in a lipid complex such as, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, Gopal, 1985). Also contemplated is a nucleic acid complexed with Lipofectamine (Gibco BRL) or Superfect (Qiagen). The amount of liposomes used may vary upon the nature of the liposome as well as the cell used, for example, about 5 to about 20 µg vector DNA/1-10×10⁶ cells may be contemplated.

Liposome-mediated nucleic acid delivery and expression of exogenous DNA in vitro has been very successful (Nicolau and Sene, 1982; Fraley et al., 1979; Nicolau et al., 1987). The feasibility of liposome-mediated delivery and expression of exogenous DNA in cultured chick embryo, HeLa and hepatoma cells has also been demonstrated (Wong et al., Gene, 10:87-94, 1980).

In certain embodiments of the present invention, a liposome may be complexed with a hemagglutinating virus (HVJ). This has been shown to facilitate fusion with the cell membrane and promote cell entry of liposome-encapsulated DNA (Kaneda et al., 1989). In other embodiments, a liposome may be complexed or employed in conjunction with nuclear non-histone chromosomal proteins (HMG-I) (Kato et al., J. Biol. Chem., 266:3361-3364, 1991). In yet further embodiments, a liposome may be complexed or employed in conjunction with both HVJ and HMG-I. In other embodiments, a delivery vehicle may comprise a ligand and a liposome.

### Electroporation

In certain embodiments of the present invention, a nucleic acid is introduced into a cell via electroporation. Electroporation involves the exposure of a suspension of cells and DNA to a high-voltage electric discharge. Recipient cells can be made more susceptible to transformation by mechanical wounding. Also the amount of vectors used may vary upon the nature of the cells used, for example, about 5 µg to about 20 µg vector DNA/1-10×10⁶ cells.

Transfection of eukaryotic cells using electroporation has been quite successful. Mouse pre-B lymphocytes have been transfected with human kappa-immunoglobulin genes (Potter et al., 1984), and rat hepatocytes have also been transfected with chloramphenicol acetyltransferase genes in this manner (TurKaspa et al., 1986).

### Calcium Phosphate

In other embodiments of the present invention, a nucleic acid is introduced into the cells using calcium phosphate precipitation. Human KB cells have been transfected with adenovirus 5DNA using this technique (Grahamand Van Der Eb, 1973). Also in this manner, mouse L(A9), mouse C127, CH0, CV-1, BHK, NIH3T3 and HeLa cells were transfected with a neomycin marker gene (Chen and Okayama, 1987), and rat hepatocytes were transfected with a variety of marker genes (Rippe et al., 1990).

### DEAE- Dextran

In other embodiments, a nucleic acid is delivered into a cell using DEAE-dextran followed by polyethylene glycol. In this manner, reporter molecule plasmids have been introduced into mouse myeloma and erythroleukemia cells (Gopal, 1985).

### Sonication Loading

Additional embodiments of the present invention include the introduction of a nucleic acid by direct sonic loading. LTK-fibroblasts have been transfected with the thymidine kinase gene by sonication loading (Fechheimer et al., 1987).

### Receptor-Mediated Transfection

In addition, a nucleic acid may be delivered to a target cell via receptor-mediated delivery vehicles. These take advantage of the selective uptake of macromolecules by receptor-mediated endocytosis that will be occurring in a target cell. In view of the cell type-specific distribution of various receptors, this delivery method adds another degree of specificity to the present invention.

Some receptor-mediated gene targeting vesicles comprise a cell receptor-specific ligand and a nucleic acid-binding agent. Others comprise a cell receptor-specific ligand to which the nucleic acid to be delivered has been operatively attached. Several ligands have been used for receptor-mediated gene transfer (Wagner et al., Proc. Natl. Acad. Sci. USA 87(9):3410-3414, 1990. Perales et al., Proc. Natl. Acad. Sci. USA, 91:4086-4090, 1994; Myers, EPO 273085), which establishes the operability of the technique. Specific delivery in the context of another mammalian cell type has been described (Wu and Wu, Adv. Drug Delivery Rev., 12:159-167, 1993). In certain aspects of the present invention, a ligand will be chosen to correspond to a receptor specifically expressed on the target cell population.

In other embodiments, a nucleic acid delivery vehicle component of a cell-specific nucleic acid targeting vesicle may comprise a specific binding ligand in combination with a liposome. The nucleic acid(s) to be delivered are housed within the liposome and the specific binding ligand is functionally incorporated into the liposome membrane. The liposome will thus specifically bind to the receptor(s) of a target cell and deliver the contents to a cell. Such systems have been shown to be functional using systems in which, for example, epidermal growth factor (EGF) is used in the receptor-mediated delivery of a nucleic acid to cells that exhibit upregulation of the EGF receptor.

In other embodiments, the nucleic acid delivery vehicle component of a targeted delivery vehicle may be a liposome itself, which will preferably comprise one or more lipids or glycoproteins that direct cell-specific binding. For example, lactosyl-ceramide, a galactose-terminal ganglioside, has been incorporated into liposomes and an increase in the uptake of the insulin gene by hepatocytes has been observed (Nicolau et al., 1987). It is contemplated that the tissue-specific transforming constructs of the present invention can be specifically delivered into a target cell in a similar manner.

### Microprojectile Bombardment

Microprojectile bombardment techniques can be used to introduce a nucleic acid into at least one organelle, cell, tissue or organism (U.S. Patent No. 5,550,318; U.S. Patent No. 5,538,880; U.S. Patent No. 5,610,042; and PCT Application WO 94/09699). This method depends on the ability to accelerate DNA-coated microprojectiles to a high velocity allowing them to pierce cell membranes and enter cells without killing them (Klein et al., Nature, 327:70-73, 1987). There are a wide variety of microprojectile bombardment techniques known in the art, many of which are applicable to the present invention.

In this microprojectile bombardment, one or more particles may be coated with at least one nucleic acid and delivered into cells by a propelling force. Several devices for accelerating small particles have been developed. One such device relies on a high voltage discharge to generate an electrical current, which in turn provides the motive force (Yang et al., 1990). The microprojectiles used have consisted of biologically inert substances such as tungsten or gold particles or beads. Exemplary particles include those comprised of tungsten, molybdenum, and preferably, gold. In some instances DNA precipitation onto metal particles would not be necessary for DNA delivery to a recipient cell using microprojectile bombardment. On the other hand, particles may contain DNA rather than be coated with DNA. DNA-coated particles may increase the level of DNA delivery via particle bombardment but are not, in and of themselves, necessary.

For the bombardment, cells in suspension are concentrated on filters or solid culture medium. Alternatively, immature embryos or other target cells may be arranged on solid culture medium. The cells to be bombarded are positioned at an appropriate distance below the microprojectile stopping plate.

In addition, in the method of the present invention, iPSCs can also be prepared by transfecting certain stem cell associated genes into non-pluripotent cells (e.g., fibroblasts) with a non-viral vector. Examples of said non-viral vector include the oligonucleotide alone or in combination with a suitable protein, polysaccharide or lipid formulation.

The term "oligonucleotide" refers to an oligomer or polymer of ribonucleic acid (RNA) or deoxyribonucleic acid (DNA) or mimetics thereof. The term "oligonucleotide" includes linear or circular oligomers of natural and/or modified monomers or linkages, including deoxyribonucleosides, ribonucleosides, substituted and alpha-anomeric forms thereof, peptide nucleic acids (PNA), linked nucleic acids (LNA), phosphorothioate, methylphosphonate, and the like. Oligonucleotides are capable of specifically binding to a target polynucleotide by way of a regular pattern of monomer-to-monomer interactions, such as Watson-Crick type of base pairing, HoOsteen or reverse HoOgsteen types of base pairing, or the like. The oligonucleotide may be "chimeric", that is, composed of different regions. "Chimeric oligonucleotides" or "chimeras," in the context of this invention, are oligonucleotides which contain two or more chemically distinct regions, each made up of at least one nucleotide. These oligonucleotides typically contain at least one region of modified nucleotides that confers one or more beneficial properties (such as, for example, increased nuclease resistance, increased uptake into cells, increased binding affinity for the RNA target) and a region that is a substrate for enzymes capable of cleaving RNA:DNA or RNA:RNA hybrids.

Non-viral vectors mainly include modified natural polymers and synthetic polymers. Modified natural polymers are mainly lipids (such as sterols), polysaccharides (such as chitosan, dextran and algal acid), proteins (such as collagen, protamine, etc.) and polypeptide substances. Synthetic polymers are mainly some biologically degradable polymers (such as polylysine, polylactic acid, polyvinyl alcohol and block or graft copolymer thereof, etc.) and cationic polymers (polyethylene imine, polyamide dendritic polymer, etc.). The modified natural polymers and synthetic polymers act as delivery vector which encapsulates DNA in the form of microspheres, vesicles or glue beam respectively and releases DNA upon arrival at the pathological site.

### Selection of iPSCs

In some aspects of the present invention, after a reprogramming vector is introduced into somatic cells, these cells will be cultured for expansion. Cells could be selected for the presence of vector elements (like reporters or selection markers) to concentrate transfected cells. Reprogramming vectors will express reprogramming factors in these cells and replicate and partition along with cell division. These expressed reprogramming factors will induce somatic cell genome to reprogram so as to establish a self-sustaining pluripotent state, and in the meantime or after removal of positive selection of the presence of vectors, exogenous genetic elements will be lost gradually. This silencing of transgene expression permits selection of induced pluripotent stem cells for tuning off reporter gene expression alone or in combination with selection for other embryonic stem cell characteristics because they are expected to be substantially identical to pluripotent embryonic stem cells.

As used herein, the term "stem cell" refers to a cell capable of self replication and having pluripotency. Typically, stem cells can regenerate an injured tissue. Stem cells herein may be, but are not limited to, embryonic stem (ES) cells or tissue stem cells (also called tissue-specific stem cell, or adult stem cell). Embryonic stem (ES) cells are pluripotent stem cells derived from early embryos. An ES cell was first established in 1981, which has also been applied to production of knockout mice since 1989. In 1998, a human ES cell was established, which is currently becoming available for regenerative medicine. Unlike ES cells, tissue stem cells have a limited differentiation potential. Tissue stem cells are present at particular locations in tissues and have an undifferentiated intracellular structure. Therefore, the pluripotency of tissue stem cells is typically low. Tissue stem cells have a higher nucleus/cytoplasm ratio and have few intracellular organelles. Most tissue stem cells have low pluripotency, a long cell cycle, and proliferative ability beyond the life of the individual. Tissue stem cells are separated into various categories, based on the sites from which the cells are derived, such as the dermal system, the digestive system, the bone marrow system, the nervous system, and the like. Tissue stem cells in the dermal system include epidermal stem cells, hair follicle stem cells, and the like. Tissue stem cells in the digestive system include pancreatic (common) stem cells, liver stem cells, and the like. Tissue stem cells in the bone marrow system include hematopoietic stem cells, mesenchymal stem cells, and the like. Tissue stem cells in the nervous system include neural stem cells, retinal stem cells, and the like.

"Induced pluripotent stem cells" used herein, commonly abbreviated as iPSCs, refer to a type of pluripotent stem cell artificially prepared from a non-pluripotent cell, typically an adult somatic cell, or terminally differentiated cell, such as fibroblast, a hematopoietic cell, a myocyte, a neuron, an epidermal cell, or the like, by inserting certain genes, referred to as reprogramming factors. "Pluripotency" used herein refers to a stem cell that has the potential to differentiate into all cells constituting one or more tissues or organs, or preferably, any of the three germ layers: endoderm (interior stomach lining, gastrointestinal tract, the lungs), mesoderm (muscle, bone, blood, urogenital), or ectoderm (epidermal tissues and nervous system). "Pluripotent stem cells" used herein refer to cells that can differentiate into cells derived from any of the three germ layers, for example, descendants of totipotent cells or induced pluripotent cells.

### Reporter Genes

Certain embodiments of the present invention utilize reporter genes to indicate successful transformation. For example, the reporter gene can be located within expression cassettes and under the control of the regulatory elements normally associated with the encoding region of a reprogramming gene for simultaneous expression. A reporter molecule allows the cells containing the reprogramming vector to be isolated without placing them under drug or other selective pressures or otherwise risking cell viability. An additional advantage is to enrich induced pluripotent stem cells with silenced reporter molecule expression.

Examples of such reporters include genes encoding cell surface proteins (e.g., CD4, HA epitope), fluorescent proteins, antigenic determinants and enzymes (e.g., galactosidase). The vector containing cells may be isolated, e.g., by FACS using fluorescently-tagged antibodies to the cell surface protein or substrates that can be converted to fluorescent products by a vector encoded enzyme.

In specific embodiments, the reporter gene is a fluorescent protein. A broad range of fluorescent protein genetic variants have been developed whose feature fluorescence emission spectral profiles span almost the entire visible light spectrum. Mutagenesis in the original Aequorea victoria jellyfish green fluorescent protein has resulted in new fluorescent probes that range in color from blue to yellow, and are some of the most widely used in vivo reporter molecules in biological research. Longer wavelength fluorescent proteins, emitting in the orange and red spectral regions, have been developed from the marine anemone, and reef corals belonging to the class Anthozoa. Still other species have been mined to produce similar proteins having cyan, green, yellow, orange, and deep red fluorescence emission. Developmental research efforts are ongoing to improve the brightness and stability of fluorescent proteins, thus improving their overall usefulness.

### Selection of iPSCs Based on Reporter Genes Expression

Cells introduced with a reprogramming factor combination in this invention could express a reporter molecule and a reprogramming factor simultaneously and selected for the presence or absence of reporter gene expression in different time points. For example, cells could be selected for the presence of reporter genes and transfection could be optimized by using reporter genes. Induced pluripotent stem cells may be screened for the loss of reporter genes as transgenes are silenced during reprogramming. Such selection or screening is based on a detectable signal generated by expression of reporter genes as an indication of transgene expression.

A detectable signal may be generated in any one of a number of ways, depending on the nature of the reporter gene employed in the method of the present invention. For example, the detectable signal may be a luminescent, such as a fluorescent signal, e.g., GFP. GFP is a fluorescent polypeptide which produces a fluorescent signal without the need for a substrate or cofactors. GFP expression and detection techniques are well known in the art, and kits are available commercially, for example from Clontech. GFP expression may be assayed in intact cells without the need to lyse them or to add further reagents. Alternatively, the detectable signal may be a signal generated as a result of enzymatic activity or the recognition of a cell surface marker, e.g., LNGFR.

Flow cytometry, for example, fluorescence-activated cell sorting (FACS), is commonly used to select detectable signals based on reporter gene expression. FACS provides a method for sorting a heterogeneous mixture of cells into two or more containers, one cell at a time, based upon the specific light scattering and fluorescent characteristics of each cell. This provides fast, objective and quantitative recording of fluorescent signals from individual cells as well as physical separation of induced pluripotent cells.

Luciferase may also be used as a basis for an assay. Luciferase expression is known in the art, and luciferase expression and detection kits are available commercially from Clontech (Palo Alto, Calif). The presence of luciferase is advantageously assessed by cell lysis and addition of luciferin substrate to the cells, before monitoring a luminescent signal by scintillation counting.

Enzyme-based assays are conducted in a manner similar to a luciferase-based assay, except that the detection is not necessarily via luminescence. The detection technique will depend on the enzyme, and may therefore be optical (such as in the case of galactosidase).

Physical and biochemical methods may also be used to identify or quantify expression of the reporter genes of the present invention. These methods include but are not limited to: 1) Southern analysis or PCR amplification for detecting and determining the structure of the recombinant DNA insert; 2) Northern blot, S-IRNase protection, primer-extension or reverse transcriptase-PCR amplification for detecting and examining RNA transcripts of the gene constructs; 3) enzymatic assays for detecting enzyme activity, where such gene products are encoded by the gene construct; 4) protein gel electrophoresis, Western blot techniques, immunoprecipitation, or enzyme-linked immunoassays, where the gene construct products are proteins; and 5) biochemical measurements of compounds produced as a consequence of the expression of the introduced gene constructs. Additional techniques, such as in situ hybridization, enzyme staining, and immunostaining, may also be used to detect the presence or expression of the reporter gene in specific cells.

### Selection for Embryonic Stem Cell Characteristics

The successfully generated iPSCs from previous studies were remarkably similar to naturally-isolated pluripotent stem cells (such as mouse and human embryonic stem cells, mESCs and hESCs, respectively) in the following respects, thus confirming the identity, authenticity, and pluripotency of iPSCs to naturally-isolated pluripotent stem cells. Thus, induced pluripotent stem cells generated from the methods disclosed in this invention could be selected based on one or more of following embryonic stem cell characteristics in addition to presence or absence of reporter gene expression.

### a. Cellular Biological Properties

Morphology: iPSCs are morphologically similar to ESCs. Each cell may have round shape, large nucleolus and scant cytoplasm. Colonies of iPSCs could be also similar to that of ESCs. Human iPSCs form sharp-edged, flat, tightly-packed colonies similar to hESCs and mouse iPSCs form the colonies similar to mESCs, less flatter and more aggregated colonies than that of hESCs.

Growth properties: Doubling time and mitotic activity are cornerstones of ESCs, as stem cells must self-renew. iPSCs could be mitotically active, actively self-renewing, proliferating, and dividing at a rate equal to ESCs.

Stem Cell Markers: iPSCs may express cell surface antigenic markers expressed on ESCs. Human iPSCs expressed the markers specific to hESC, including, but not limited to, SSEA-3, SSEA-4, TRA-1-60, TRA-1-81, TRA-2-49/6E, and Nanog. Mouse iPSCs express SSEA-1 but not SSEA-3 nor SSEA-4, similarly to mESCs.

Stem Cell Genes: iPSCs may express genes expressed in undifferentiated ESCs, including Oct-3/4, Sox2, Nanog, GDF3, REX1, FGF4, ESG1, DPPA2, DPPA4, and hTERT.

Telomerase Activity: Telomerases are necessary to sustain cell division unrestricted by the Hayflick limit of ∼50 cell divisions. ESCs express high telomerase activity to sustain self-renewal and proliferation, and iPSCs also demonstrate high telomerase activity and express TERT (human telomerase reverse transcriptase), a necessary component in the telomerase protein complex.

Pluripotency: iPSCs will be capable of differentiation in a fashion similar to ESCs into fully differentiated tissues.

Neural Differentiation: iPSCs can be differentiated into neurons, expressing β III-tubulin, tyrosine hydroxylase, AADC, DAT, ChAT, LMX1B, and MAP2. The presence of catecholamine-associated enzymes may indicate that iPSCs, like hESCs, may be differentiated into dopaminergic neurons. Stem cell-associated genes will be down-regulated after differentiation.

Cardiac Differentiation: iPSCs can be differentiated into cardiomyocytes that spontaneously begin beating. Cardiomyocytes express TnTc, MEF2C, MYL2A, MYHC0 and NKX2.5. Stem cell-associated genes will be down-regulated after differentiation.

Teratoma Formation: iPSCs injected into immunodeficient mice may spontaneously form teratomas after certain time, such as nine weeks. Teratomas are tumors of multiple lineages containing tissue derived from the three germ layers (endoderm, mesoderm and ectoderm); this is unlike other tumors, which typically are of only one cell type. Teratoma formation is a landmark test for pluripotency.

Embryoid Body: ESCs in culture spontaneously form ball-like embryo-like structures termed as "embryoid bodies", which consist of a core of mitotically active and differentiating ESCs and a periphery of fully differentiated cells from all three germ layers. iPSCs may also form embryoid bodies and have peripheral differentiated cells.

Blastocyst Injection: ESCs naturally reside within the inner cell mass (embryoblast) of blastocysts, and in the embryoblast, differentiate into the embryo while the blastocyst's shell (trophoblast) differentiates into extraembryonic tissues. The hollow trophoblast is unable to form a living embryo, and thus it is necessary for the embryonic stem cells within the embryoblast to differentiate and form the embryo. iPSCs injected by micropipette into a trophoblast to generate a blastocyst transferred to recipient females, may result in chimeric living mouse pups: mice with iPSC derivatives incorporated all across their bodies with 10%-90% chimerism.

### b. Epigenetic reprogramming

Promoter Demethylation: Methylation is the transfer of a methyl group to a DNA base, typically the transfer of a methyl group to a cytosine molecule in a CpG site (adjacent cytosine/guanine sequence). Widespread methylation of a gene interferes with expression by preventing the activity of expression proteins or recruiting enzymes that interfere with expression. Thus, methylation of a gene effectively silences it by preventing transcription. Promoters of endogenous pluripotency-associated genes, including Oct-3/4, Rex1, and Nanog, may be demethylated in iPSCs, showing their promoter activity and the active promotion and expression of pluripotency-associated genes in iPSCs.

Histone Demethylation: Histones are compacting proteins that are structurally localized to DNA sequences that can effect their activity through various chromatin-related modifications. H3 histones associated with Oct-3/4, Sox2, and Nanog may be demethylated to activate the expression of Oct-3/4, Sox2, and Nanog.

### Culturing of iPSCs

During the preparation of induced pluripotent stem cells in accordance with the method of the invention, induction reprogramming factors and the selection marker are expressed in the stage of cell culturing, after which the obtained cells are selected for the aforementioned cell biology properties and epigenetic properties. In some embodiments, the cells may be cultured for a period of time prior to the induction process. Alternatively, the cells may be induced and selected directly without a prior culture period. In some embodiments, different cell culture media are used at different points. For example, one type of culture medium may be used before the induction process, while a second type of culture medium is used during the induction process. At times, a third type of culture medium is used during the selection process.

After collecting cells from donor samples such as tissue or cellular samples, a suitable medium can be used according to different cell types or tissue types. Some representative media include but are not limited to: multipotent adult progenitor cell (MAPC) medium; FBM (manufactured by Lonza); embryonic stem cell (ESC) medium; mesenchymal stem cell growth medium (MSCGM) (manufactured by Lonza); MCDB202 modified medium; endothelial cell medium kit-2 (EBM2) (manufactured by Lonza); IMDM; DMEM; MEF-conditioned ES; and mTeSR™.

In this invention, the culture media used for the induction of iPSs are mainly as follows:

mES culture medium: DMEM (High Glucose, Gibco) + 15%FBS (Gibco) + 1×non-essential amino acid + 1×glutamine GlutaMAX (1×, Gibco) + sodium pyruvate (1×, Gibco) + penicillin/ streptomycin (50untis/ml P, 50mg/ml S, Hyclone) + 0.1mM β-mercaptoethanol +1000u leukocyte inhibitory factor LIF (millipore).

Serum-free culture medium iSF1: the components contained in the medium and the amounts thereof have been disclosed in Chinese Patent Application No. CN200910038883.4 (PCT/CN2009/074358).

Chemically defined serum-free culture medium iCD1: the components contained in the medium and the amounts thereof have been disclosed in Chinese Patent Application No. 201010167062.3 and Rational optimization of reprogramming culture conditions for the generation of induced pluripotent stem cells with ultra-high efficiency and fast kinetics. Cell Res 21, 884-894.

MEF culture medium: comprising high glucose DMEM, 2mM L-glutamine, 1×non-essential amino acid.

ES culture medium: including the following types:
(1) mES culture medium: DMEM (High Glucose, Gibco) + 15%FBS (Gibco) + 1×non-essential amino acid + 2mM glutamine GlutaMAX (1×, Gibco) + sodium pyruvate (1×, Gibco) + penicillin/ streptomycin (50untis/ml P, 50mg/ml S, Hyclone) + 0.1mM β-mercaptoethanol +1000u leukocyte inhibitory factor (LIF) (millipore);
(2) mKSR culture medium: knock-out DMEM (Gibco) + 15%KSR + 1×non-essential amino acid + 2mM glutamine GlutaMAX (1×, Gibco) + penicillin/streptomycin (50 untis/ml P, 50mg/ml S, Hyclone) + 0.1mM β-mercaptoethanol +1000u leukocyte inhibitory factor (LIF) (millipore);
(3) N2B27+2i culture medium: knock-out DMEM (Gibco)/DMEM (High Glucose, Gibco) (1/1) + N2 without serum additives (Gibco, 0.5X) + B27 without serum additives (Gibco, 0.5X) + 1×non-essential amino acid + 2mM glutamine GlutaMAX (1×, Gibco) + penicillin/streptomycin (50untis/ml P, 50mg/ml S, Hyclone) + 0.1mM β-mercaptoethanol + 1000u leukocyte inhibitory factor LIF (millipore) + 3µM CHIR99021+1µM PD0325901.

Primary culture occurs immediately after the cells are isolated from a donor, e.g., human or mouse. The cells can also be sub-cultured after the primary culture. A "second" subculture describes subculturing primary culture cells once. In some cases, the primary cells are subjected to a subculture once (i.e., second subculture), or twice (i.e., third subculture). The culture techniques are well-known in the art. In the preferred embodiments of the present invention, cells are cultured from the primary culture to the second subculture. In some cases, the cells may be cultured for 1 to 12 days e.g., 2 days, 3 days, 4 days, 5 days, 6 days, 7 days, 8 days, 9 days, 10 days, and so on prior to induction. In other cases, the cells may be cultured for more than 12 days, e.g. from 12 days to 20 days; from 12 days to 30 days; or from 12 days to 40 days. In some embodiments, the cells to be induced are passaged for example one, two or three times prior to induction.

In some cases, cells are cultured at a low density, e.g., 1×10³ cells/cm² or 1×10⁴ cells/cm². In other cases, cells are cultured at a density e.g., 1×10³ cells/cm² to 3×10⁴ cells/cm².

Often the cells and/or tissues are cultured in a first medium, prior to the introduction of induction reprogramming factors into the cells; and then the cells are cultured in a second or third medium during and/or after the introduction of the induction reprogramming factors into the cells.

In some embodiments of the present invention, the cells are cultured in MEF culture medium immediately when a retroviral transfection period begins; and then, following the initiation of expression of induction reprogramming factors, the cells are cultured in mES, mES+Vc, iSF1, iCD1 and other ES cell culture media.

When a culture medium of either a low or high serum concentration is used for culturing the cells, one or more growth factors such as fibroblast growth factor (basic FGF); platelet-derived growth factor (PDGF), epidermal growth factor (EGF); insulin-like growth factor (IGF); IGF II; or insulin can be included in the culture medium. Other growth factors that can be used to supplement cell culture media include, but are not limited to: Transforming Growth Factor β-1 (TGF β-1 ), Activin A, Brain-derived Neurotrophic Factor (BDNF), Nerve Growth Factor (NGF), Neurotrophin (NT)-1, NT-2, or NT-3. In some embodiments, one or more of such factors is used in place of the bFGF or FGF-2 in the MC-ES medium or other cell culture medium.

The concentration of growth factors (e.g., bFGF, EGF, IGF, insulin, TGF β-1, Activin A) in the culture media described herein (e.g., mES, iSF1, iCD1, N2B27+2i, mTeSRl™) may be from 4 ng/ml to 50 ng/ml, e.g., 2 ng/ml, 3 ng/ml, 4 ng/ml, 5 ng/ml, 6 ng/ml, 7 ng/ml, 8 ng/ml, 10 ng/ml, 12 ng/ml, 14 ng/ml, 15 ng/ml, 17 ng/ml, 20 ng/ml, 25 ng/ml, 30 ng/ml, 35 ng/ml, 40 ng/ml, 45 ng/ml, or 50 ng/ml.

In the specific embodiment of the present invention, the iPSCs can be maintained in the following culture media like the mouse embryonic stem cells (mESCs):
(1) DMEM (High Glucose, Gibco) + 15%FBS (Gibco) + 1×non-essential amino acid + 2mM glutamine GlutaMAX (1×, Gibco) + sodium pyruvate (1×, Gibco) + penicillin/streptomycin (50untis/ml P, 50mg/ml S, Hyclone) + 0.1mM β-mercaptoethanol +1000u leukocyte inhibitory factor LIF (millipore);
(2) N2B27+2i culture medium: knock-out DMEM (Gibco)/DMEM (High Glucose, Gibco) (1/1) + N2 without serum additives (Gibco, 0.5X) + B27 without serum additives (Gibco, 0.5X) + 1×non-essential amino acid + 2mM glutamine GlutaMAX (1×, Gibco) + penicillin/streptomycin (50untis/ml P, 50mg/ml S, Hyclone) + 0.1mM β-mercaptoethanol + 1000u leukocyte inhibitory factor LIF (millipore) + 3µM CHIR99021 + 1µM PD0325901.

In the method for preparing iPSCs of the present invention, the cells to be induced may be cultured in a 37⁰C, 5%CO₂ incubator with medium changes preferably every day. In some embodiments, the cells are cultured for 8 days to 15 days, prior to identifying and selecting induced pluripotent stem cell colonies based on cell biological properties and epigenetic properties as described herein.

### Uses of iPSCs Prepared by the Invention

iPSCs have many uses. They may be further differentiated to generate different types of cells, e.g., neurons, hepatocytes, or cardiomyocytes. They may also give rise to other types of stem cells, e.g., neural stem cells, hepatic stem cells, or cardiac stem cells They have the ability to differentiate into other cells of a specific lineage. The induced cells, and cells differentiated from them, are useful for cell transplantation therapies. Since the induced cells can be induced from non-embryonic cells, a cell transplantation therapy can involve providing a subject with cells derived from his or her own tissue, thereby lessening the possibility of immune rejection.

iPSCs can also be used for screening chemotherapeutic drugs. iPSCs obtained by the method of the present invention are further differentiated into cells of a specific cell line, such as neurons, hepatocytes, cardiomyocytes, epithelial cells, and so on. Then the drugs to be screened are applied to various types of cells for the determination of cytotoxicity and efficacy, etc. of drugs to be screened, thereby screening the drugs with the lowest cytotoxicity and the highest efficacy.

### Differentiation of iPSCs

iPSCs may be differentiated into various different types of lineages. Examples of differentiated cells include differentiated cells from ectodermal (e.g., neurons and fibroblasts), mesodermal (e.g., cardiomyocytes), or endodermal (e.g., pancreatic cells) lineages. The differentiated cells may be one or more of the following cells: pancreatic β-cells, neural stem cells, neurons (e.g., dopaminergic neurons), oligodendrocytes, oligodendrocyte progenitor cells, hepatocytes, hepatic stem cells, astrocytes, cardiomyocytes, hematopoietic cells, or cardiac cells.

The differentiated cells derived from the induced pluripotent stem cells prepared by the method of the present invention may be terminally differentiated cells, or they may be capable of giving rise to cells of a specific lineage. For example, iPSCs can be differentiated into a variety of multipotent cell types, e.g., neural stem cells, cardiac stem cells, or hepatic stem cells. The stem cells may then be further differentiated into new cell types, e.g., neural stem cells may be differentiated into neurons; cardiac stem cells may be differentiated into cardiomyocytes; and hepatic stem cells may be differentiated into hepatocytes.

Numerous methods for differentiating iPSCs into cells of a specific cell type are well known in the art. Methods for differentiating iPSCs may be similar to those used to differentiate stem cells, such as embryonic stem cells, hematopoietic stem cells and so on. In some cases, the differentiation occurs in vitro; and in some cases the differentiation occurs in vivo.

Methods for generating neural stem cells from embryonic stem cells through differentiation as well known to those skilled in the art may be used to generate neural stem cells from iPSCs, see, e.g., Reubinoff et al., Nat, Biotechnol., 19(12): 1134-40, 2001, Nat, Biotechnol., 19(12):1134-40. For example, neural stem cells may be generated by culturing iPSCs in suspension in the presence of growth factors, e.g., FGF-2, see, Zhang et al., Nat. Biotech., (19):1129-1133, 2001. In some cases, the aggregates of iPSCs are cultured in a serum-free culture medium containing FGF-2. In another embodiment, iPSCs may be co-cultured with a mouse stromal cell line in the presence of a serum-free culture medium comprising FGF-2. In yet another embodiment, iPSCs may be directly transferred to a serum- free culture medium containing FGF-2 to directly induce differentiation of iPSCs.

Neural stem cells derived from iPSCs may be further differentiated into neurons, oligodendrocytes, or astrocytes. Often, the conditions used to generate neural stem cells can also be used to generate neurons, oligodendrocytes, or astrocytes.

iPSCs may be further differentiated into pancreatic β-cells by methods known in the art, e.g., Lumelsky et al., Science, 292:1389-1394, 2001; Assady et al., Diabetes, 50:1691-1697, 2001; D'Amour et al., Nat. Biotechnol, 24:1392-1401, 2006; D'Amour et al., Nat. Biotechnol, 23:1534-1541, 2005. The method comprises culturing iPSCs in a serum-free culture medium supplemented with Activin A, followed by culturing iPSCs in a serum-free culture medium supplemented with all-trans retinoic acid, followed by culturing iPSCs in a serum-free culture medium supplemented with bFGF and nicotinamide.

Hepatic cells or hepatic stem cells may be differentiated from iPSCs. For example, culturing iPSCs in the presence of sodium butyrate may generate hepatocytes, see e.g., Rambhatla et al., Cell Transplant, 12:1-11, 2003. In some embodiments, iPSCs may be differentiated into hepatic cells or hepatic stem cells by culturing iPSCs in the presence of Activin A for 2 to 6 days, e.g., 2, 3, 4, 5, or 6 days, and then culturing iPSCs in the presence of hepatocyte growth factor for 5 days to 10 days, e.g., 5, 6, 7, 8, 9, or 10 days.

### Composition

The invention also provides a composition for promoting the formation of a pluripotent stem cell, said composition comprising:
(i) a c-Jun antagonist, and one factor selected from the following seven groups of factors : (1) Sox2, Klf4 and c-Myc, (2) Klf4 and c-Myc, (3) Oct3/4, Klf4 and c-Myc, (4) Sox2, Nanog and Lin28, (5) Oct3/4, Nanog and Lin28, (6) Oct3/4, Klf and Sox2, and (7) Klf4 and Sox2; or
said composition comprising:
(ii) a c-Jun antagonist, Jhdmlb and Id1; at least one of Glis1, Sall4 and Lrhl; or
said composition comprising:
(iii) a c-Jun antagonist, Jhdmlb and Id1; and at least one of Oct4, Klf4, Sox2, Lin28, Esrrb, Lefl, Utfl and miRNA C.

In some embodiments of the present invention, the composition for promoting the formation of an induced pluripotent stem cell of the present invention comprises: a c-Jun antagonist and Sox2, Klf4 and c-Myc, or, the composition comprises a c-Jun antagonist and Oct3/4, Klf4 and c-Myc, or, the composition comprises a c-Jun antagonist and Oct3/4, Klf4 and c-Myc, or, the composition comprises a c-Jun antagonist and Oct3/4, Klf and Sox2, or, the composition comprises a c-Jun antagonist and Sox2 and Klf4.

In one embodiment of the present invention, the composition for promoting the formation of an induced pluripotent stem cell of the present invention comprises: a c-Jun antagonist, Jhdmlb and Id1; and at least one of Glis1, Sall4 and Lrhl. In a preferred embodiment, the composition comprises a c-Jun antagonist, Jhdmlb and Id1; and Glis1, Sall4 or Lrhl.

In some embodiments of the present invention, the composition for promoting the formation of an induced pluripotent stem cell of the present invention comprises: a c-Jun antagonist, Jhdmlb and Id1 as well as Oct4; or, the composition comprises: a c-Jun antagonist, Jhdmlb and Id1 as well as Klf4; or, the composition comprises: a c-Jun antagonist, Jhdmlb and Id1 as well as Sox2; or, the composition comprises: a c-Jun antagonist, Jhdmlb and Id1 as well as Lin28; or, the composition comprises: a c-Jun antagonist, Jhdmlb and Id1 as well as Esrrb; or, the composition comprises: a c-Jun antagonist, Jhdmlb and Id1 as well as Lefl; or, the composition comprises: a c-Jun antagonist, Jhdmlb and Id1 as well as Utfl; or, the composition comprises: a c-Jun antagonist, Jhdmlb and Id1 as well as miRNA C.

In some embodiments, the composition of the invention can be introduced into cells (e.g., mouse embryonic fibroblasts) obtained from a donor (such as mouse or human) for example by a retroviral or lentiviral vector, and then the cells are cultured under the condition for culturing iPSCs as described herein, to reprogram somatic cells in vitro, thereby preparing iPSCs.

In some embodiments, the composition of the present invention can comprise one or more kinds of viruses transfected with the factor of the invention capable of promoting reprogramming and a virus transfected with a c-Jun antagonist, respectively, wherein said virus is for example a retrovirus, a lentivirus, a pox virus, an adenovirus, and so on. In some embodiments, the composition of the present invention can comprise one or more nucleic acids which encode the factor of the present invention capable of promoting reprogramming and a nucleic acid transfected with a c-Jun antagonist. In some embodiments, the composition of the present invention can comprise a liposome encapsulating one or more viral vectors, wherein said viral vectors are transfected with the factor of the present invention capable of promoting reprogramming and a c-Jun antagonist, respectively.

### Kit

The invention further provides a kit comprising a composition for promoting the formation of an induced pluripotent stem cell, wherein said kit further comprises a package and/or instructions for use of the composition of the present invention. Said kit further comprises a culture plate, a culture medium (for example, ES culture medium, MC-ES culture medium) and a supplement (e.g., fibroblast growth factor (FGF) -2, basic FGF, platelet-derived growth factor (PDGF), epithelial growth factor (EGF) and insulin) required for culturing an induced pluripotent stem cell by using the composition of the present invention, as well as a green fluorescent protein (GFP) capable of indicating the formation of iPSCs. The kit may also include a tool for collecting a cell or tissue sample from a donor, a culture flask for the preservation of the cell or tissue sample, etc. The instructions contained in the kit can provide users with uses of the composition and related information. The instructions can be of any suitable forms, including, but not limited to, publications, videos, computer readable discs or compact discs.

### Examples

### Example 1. Inhibitory effect of c-Jun on somatic cell reprogramming

### (1) c-Jun plays different roles in somatic cells and stem cells

In order to study the roles of c-Jun played in somatic cells and stem cells, mouse embryonic stem cells and mouse embryo fibroblasts were selected as models and the total mRNAs were extracted from mouse embryonic stem cells and mouse embryonic fibroblasts using TRIzol. Subsequently, qPCR was carried out using the following steps: cDNAs were prepared with ReverTra Ace (Toyobo) and oligo-dT, and then qPCR (Takara) assay was conducted with Premix Ex Taq.

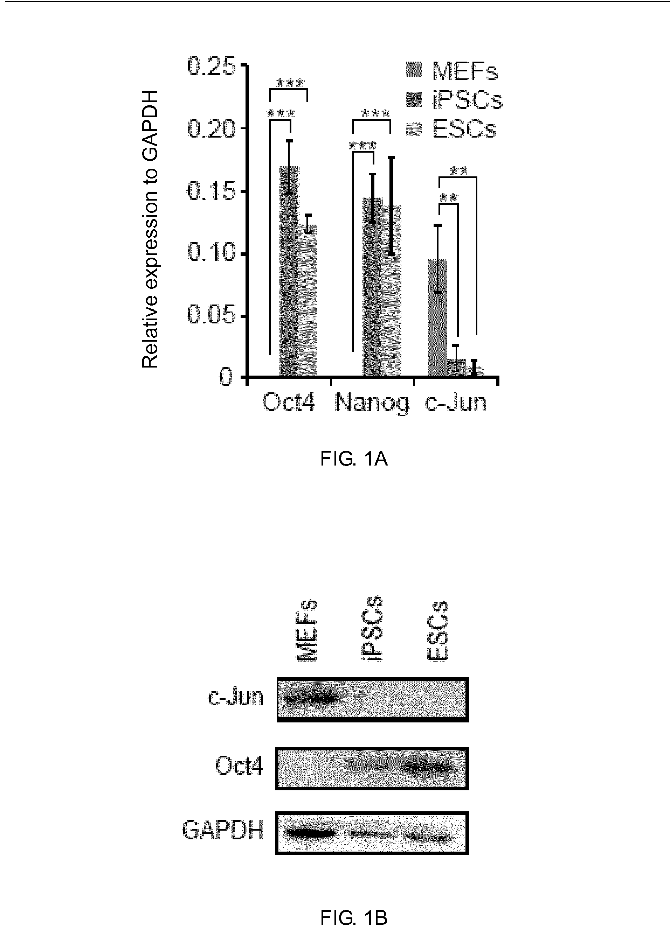

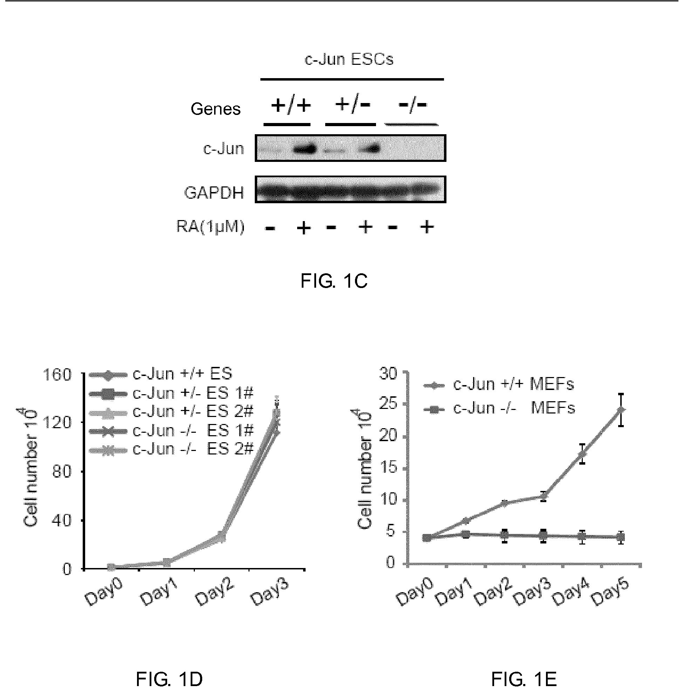

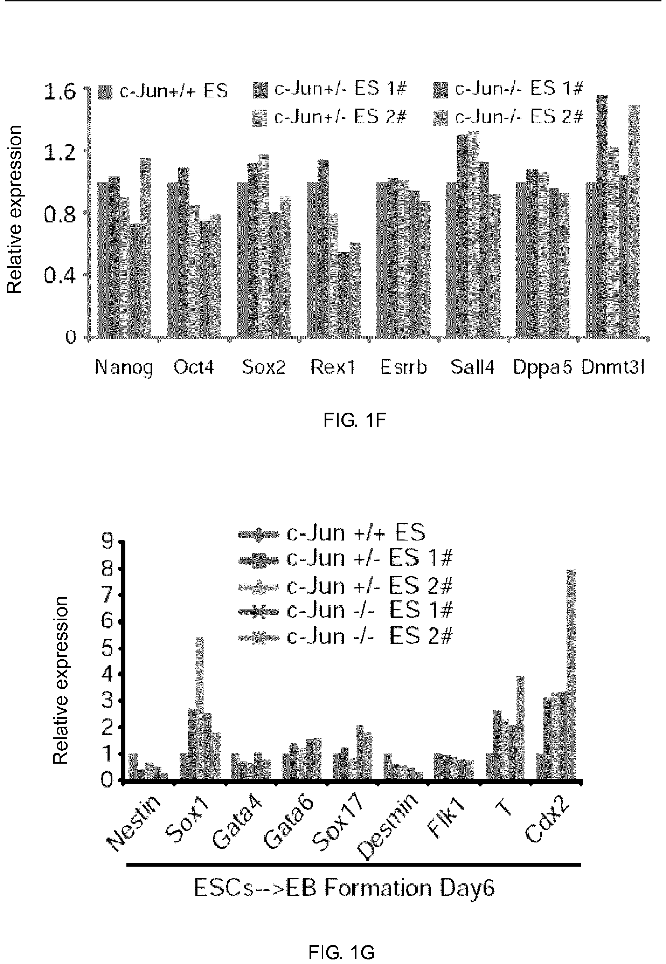

FIG. 1A shows expression levels of c-Jun in mouse embryonic stem cells, induced pluripotent stem cells and mouse embryonic fibroblasts, compared with Oct4 and Nanog. As shown in FIG. 1A, c-Jun was weakly expressed in mouse embryonic stem cells and induced pluripotent stem cells, while highly expressed in mouse embryo fibroblasts.

In order to further verify the results obtained by qPCR, Western blot assay was performed as follows (with GAPDH as control): cell cultures were lysed using a protein lysate, followed by SDS-PAGE gel electrophoresis separation. The resultant proteins were transferred to a membrane and the membrane was blocked and then incubated with a primary antibody corresponding to the protein of interest. The membrane was further incubated with a corresponding secondary antibody. Finally, the membrane was developed with a developer and X-ray film was exposed to the membrane and protein expression amount was observed by the size of a specific band. FIG. 1B shows expression levels of c-Jun in mouse embryonic stem cells, induced pluripotent stem cells and mouse embryonic fibroblasts, compared with Oct4. As shown in FIG. 1B, c-Jun was weakly expressed in mouse embryonic stem cells and induced pluripotent stem cells, while highly expressed in mouse embryonic fibroblasts, consistent with the results obtained by the above qPCR assay.

Next, gene knockout was performed on c-Jun through the following steps: by using TALEN gene editing techniques, firstly, a c-Jun gene site-specific TALEN vector was designed, and at the same time a Jun-deficient plasmid having puromycin resistance was constructed to replace c-Jun in genome, and homozygous knockout c-Jun (c-Jun^{-/-}) and heterozygous knockout c-Jun (c-Jun^{+/-}) were finally obtained through resistance screening and gene identification and verified at the protein level (as shown in FIG. 1C). The expression levels of c-Jun in wild-type c-Jun (c-Jun^{+/+}), c-Jun^{-/-} and c-Jun^{+/-} mouse embryonic stem cells and the influences of the above-mentioned gene knockout on the proliferation of mouse embryonic stem cells and mouse fibroblasts were compared. FIGS. ID and IE show changes in cell number of c-Jun^{+/+}, c-Jun^{-/-} and c-Jun^{+/-} cells with the increase of cell culture days. As shown in FIG. ID, number of c-Jun^{+/+}, c-Jun^{-/-} and c-Jun^{+/-} mouse embryonic stem cells increased significantly, while number of c-Jun^{-/-} mouse embryonic fibroblasts did not increase, and only number of c-Jun^{+/+} mouse embryonic fibroblasts increased (as shown in FIG. IE). Furthermore, as shown in FIGS. IF and 1G, in c-Jun^{-/-} mouse embryonic stem cells, the expression levels of pluripotent genes were similar, and c-Jun^{-/-} mouse embryonic stem cells were able to differentiate into cells of three germ layers in EB ball experiment. These results indicate that c-Jun homozygous knockout c-Jun^{-/-} mouse embryonic stem cells proliferated normally, while c-Jun^{-/-} mouse fibroblasts failed to proliferate normally. It is shown that c-Jun expressed differently and played different roles in somatic cells and stem cells.

In the context of the present invention, the primers (5'-3') used in the PCR analysis were as follows:

| | |
|---|---|
| mFos-q-F | GGGAACGGAATAAGATGGCT (SEQ ID NO.4) |
| mFos-q-R | TGGGCTGCCAAAATAAACTC (SEQ ID NO.5) |
| mFosB-q-F | TGTCTTCGGTGGACTCCTTC (SEQ ID NO.6) |
| mFosB-q-R | GATCCTGGCTGGTTGTGATT (SEQ ID NO.7) |
| mFra1-q-F | GAGACCGACAAATTGGAGGA (SEQ ID NO.8) |
| mFra 1-q-R | CTCCTTCTGGGATTTTGCAG (SEQ ID NO.9) |
| mFra2-q-F | CCTTGTCTTCACCTACCCCA (SEQ ID NO.10) |
| mFra2-q-R | TCCCCACTGCTACTGCTTCT (SEQ ID NO.11) |
| mATF2-q-F | CAAGAAGGCTTCCGAAGATG (SEQ ID NO.12) |
| mATF2-q-R | AGGTAAAGGGCTGTCCTGGT (SEQ ID NO.13) |
| mATF3-q-F | ACAACAGACCCCTGGAGATG (SEQ ID NO.14) |
| mATF3-q-R | TCTTGTTTCGACACTTGGCA (SEQ ID NO.15) |
| mJun-q-F | TCCCCTATCGACATGGAGTC (SEQ ID NO.16) |
| mJun-q-R | GAGTTTTGCGCTTTCAAGGT (SEQ ID NO.17) |
| mJdp2-q-F | AGAAGAGCGAAGGAAAAGGC (SEQ ID NO.18) |
| mJdp2-q-R | CTCTGCAGAAACTCTGTGCG (SEQ ID NO.19) |
| GAPDH-q-f | AACTTTGGCATTGTGGAAGGGCTCA (SEQ ID NO.20) |
| GAPDH-q-r | TTGGCAGCACCAGTGGATGCAGGGA (SEQ ID NO.21) |
| Nanog-q-f | CTCAAGTCCTGAGGCTGACA (SEQ ID NO.22) |
| Nanog-q-r | TGAAACCTGTCCTTGAGTGC (SEQ ID NO.23) |
| Dppa5-q-f | CCGTGCGTGGTGGATAAG (SEQ ID NO.24) |
| Dppa5-q-r | GCGACTGGACCTGGAATAC (SEQ ID NO.25) |
| Rex1-q-f | CAGCCAGACCACCATCTGTC (SEQ ID NO.26) |
| Rex1-q-r | GTCTCCGATTTGCATATCTCCTG (SEQ ID NO.27) |
| mDnmt31-q-f | CGGAGCATTGAAGACATC (SEQ ID NO.28) |
| mDnmt31-q-r | CATCATCATACAGGAAGAGG (SEQ ID NO.29) |
| Sox2-q-f | AGGGCTGGGAGAAAGAAGAG (SEQ ID NO.30) |
| Sox2-q-r | CCGCGATTGTTGTGATTAGT (SEQ ID NO.31) |
| Errsb-q-f | TTTCTGGAACCCATGGAGAG (SEQ ID NO.32) |
| Errsb-q-r | AGCCAGCACCTCCTTCTACA (SEQ ID NO.33) |
| Sall4-q-r | CTAAGGAGGAAGAGGAGAG (SEQ ID NO.34) |
| Sall4-q-r | CAAGGCTATGGTCACAAG (SEQ ID NO.35) |
| Oct4-q-f | CATTGAGAACCGTGTGAG (SEQ ID NO.36) |
| Oct4-q-r | TGAGTGATCTGCTGTAGG (SEQ ID NO.37) |

### (2) Inhibitory effect of c-Jun on somatic cell reprogramming

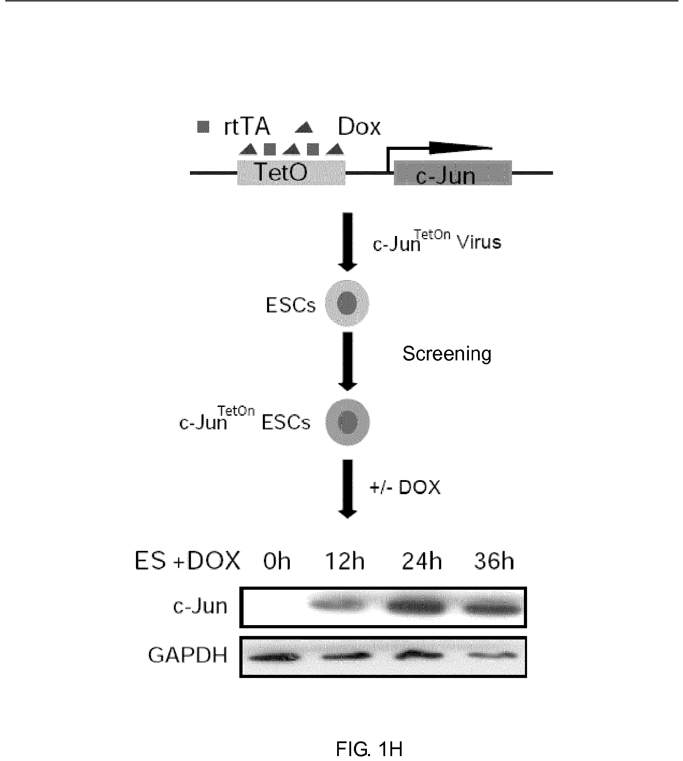

As shown in FIG. 1H, mouse embryonic stem cells (c-Jun^{TetOn} ESC) for DOX inductively expressing c-Jun were established by the following method to study the influence of c-Jun on the differentiation of mouse embryonic stem cells: c-Jun gene nucleotide sequence was cloned to pW-TRE inducible lentiviral expression vector, and embryonic stem cells were infected with a packaged virus. Different embryonic stem cells were selected and after passage were cultured in embryonic stem cell culture medium with or without the addition of DOX, respectively. RNA and protein samples were collected and q-PCR and Western blot assay were performed to identify whether ES clones were imported into c-Jun induction.

Mouse somatic cell reprogramming was performed in the following manner:

### I. Cells were cultured using the following culture media:

The culture medium for feeder layer cells, mouse embryonic fibroblasts and PlatE cells consists of: high glucose basal medium DMEM, plus 10% fetal bovine serum (FBS).

The culture medium for mouse embryonic stem cells consists of: DMEM high glucose medium, 15% FBS, 1% NEAA (GIBCO), 1mM L-glutamine, 0.1mM β-mercaptoethanol, and leukocyte inhibitory factor (LIF).

iCD1 culture medium: the components contained in the medium and the amounts thereof have been disclosed in Chinese Patent Application No. CN201010167062.3.

KSR (Knockout Serum Replace) serum-free culture medium is a commercialized serum replacing stem cell culture additive and is used as a complete KSR serum-free medium for culturing stem cells or iPSCs, the composition of which consists of: Knockout-DMEM, 15% kKSR, 1% NEAA (GIBCO), 1mM L-glutamine, 0.1mM β-mercaptoethanol and leukocyte inhibitory factor (LIF).

### II. Cells for reprogramming

All the somatic cells for reprogramming are OG2 mouse embryonic fibroblasts (lab-made), the passage number of which does not exceed three. One property of the OG2 mouse is that there is connected a green fluorescence protein (GFP) behind the promoters of the gene Oct4 that is specifically expressed by the stem cells. In reprogramming, when the endogenous Oct4 of the OG2 mouse embryonic fibroblasts is activated, the green fluorescence protein is expressed concomitantly. As observed through a fluorescence microscope, the successfully reprogrammed cells or cloned cell aggregates are green, and it is easy to compare the reprogramming efficiency at different conditions by directly adding up the number of reprogrammed clones, i.e., the number of green fluorescence clones, or by analyzing the proportion of green fluorescence cells through a flow cytometer.

### III. Method for inducing somatic cell reprogramming

The reprogrammed cells were prepared as follows. The cells were seeded in 12-well plates at a density of 20000 cells per well, and after 8 hours, were infected with viruses with the mouse reprogramming factor

### i. Preparation of viruses

The transcription factors for reprogramming include the retrovirus vector pMXs for cDNAs of mouse Oct4, Sox2, Klf4, and c-Myc; Oct4, NCBI accession number: NM_013633.2; Sox2, NCBI accession number: NM_011443.3; Klf4, NCBI accession number: NM_010637.2; and c-Myc, NCBI accession number: NM_010849.4. The reprogramming factor plasmids on the pMX vector were transfected into the viral packaging cells (PlatE) by using a transfection reagent commonly used in the art, and 48 hours after the transfection, the virus supernatant was collected with a syringe and filtered into an appropriate centrifuge tube with a 0.45µm filter membrane to be used as the viral solution for first infection; a fresh 10 ml 10% FBS culture medium was added to the plat-E culture plate (100mm plate), and the plate was placed in a 5% CO₂, 37°C incubator to continue the culture. After 24 hours, the virus was recollected in the same way to be used for second infection.

### ii. Infecting mouse embryonic fibroblasts with viruses

Infection was conducted in two rounds. Viral supernatant collected each time was mixed with MEF cell culture medium in a ratio of 1:1 (the ratio of Oct4:Sox2:Klf4:c-Myc:substrate culture medium was 1:1:1:1:1 for a four-factor infection, and the ratio of Oct4:Sox2:Klf4:substrate culture medium was 1:1:1:1 for a three-factor infection, and so on), a final concentration of 8µg/ml hexadimethrine bromide (polybrene) was added and the resultant mixture was evenly mixed. Then the first round of infection was carried out on the MEF cells, and after 24 hours the second round of infection was carried out on the re-collected virus in the same way. At different time points after infection, the cells with GFP fluorescence were observed with a fluorescence microscope.

iii. The proportion of GFP fluorescence cells was analyzed using a flow cytometer or the infected cells were cultured until formation of induced pluripotent stem cell clones. Typical iPS clones were morphologically similar to embryonic stem cells and displayed green fluorescence (using reporter cells with GFP). The number of clones with GFP fluorescence was add up to compare the reprogramming efficiency.

According to the method for the formation of stem cell clones as described above, various factor combinations for promoting the formation of induced pluripotent stem cells within the scope of the present invention can be used to carry out the experiments.

In the context of the present invention, illustrative factor combinations that promote the formation of induced pluripotent stem cells formation were provided as follows:
(1) Oct4, Klf4 and Sox2, abbreviated as OKS system,
(2) Oct4, c-Myc, Klf4 and Sox2, abbreviated as OKSM system,
(3) Klf4, Sox2 and c-Myc, abbreviated as KSM system,
(4) Klf4 and Sox2, abbreviated as KS system,
(5) c-JunDN/Jdp2 and Jhdm1b, as well as Id1/3, Glis1, Sall4, and Lrh1, abbreviated as 6F system.

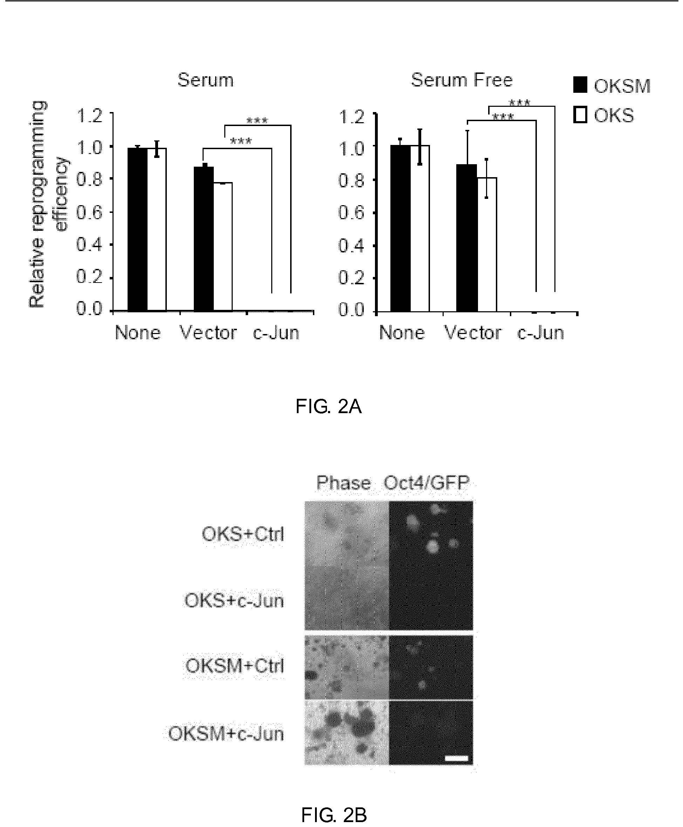

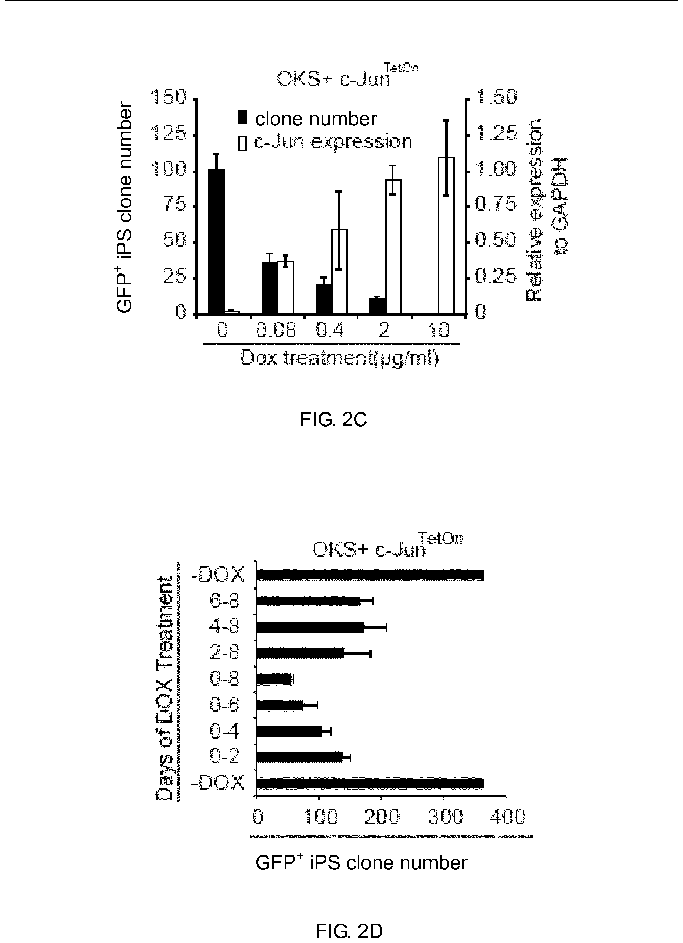

The influences of c-Jun on the reprogramming efficiency of somatic cells in OKS system and OKSM system were examined by using the above-mentioned method. FIG. 2A shows clone number of induced pluripotent stem cells growing in a serum-containing culture medium and a serum-free culture medium in c-Jun+OKS system and c-Jun+OKSM system, and FIG. 2B shows micrographs of induced pluripotent stem cells obtained in c-Jun+OKS system, control+OKS system, c-Jun+OKSM system, and OKSM+control system, wherein the controls are OKS and OKSM systems without the addition of c-Jun. As shown in FIGS. 2A and 2B, c-Jun strongly inhibited the reprogramming of somatic cells, the reprogramming efficiency when using c-Jun was almost zero and in the microscopic field almost no induced pluripotent stem cells were observed. Subsequently, changes in expression levels of c-Jun and GFP⁺ iPS clone numbers with the change of the concentration of DOX in c-Jun^{TetOn} ESC+OKS system were examined. FIG. 2C shows expression levels of c-Jun and GFP⁺ iPS clone numbers under the conditions of different DOX concentrations. It can be seen that without the addition of DOX, the expression level of c-Jun was almost zero and GFP⁺ iPS clones had the biggest number; with the continuous addition of DOX, the expression levels of inducible c-Jun enhanced, while GFP⁺ iPS clone numbers decreased gradually, which suggests that c-Jun inhibited the reprogramming in a dose-dependent manner. At the same time, changes in expression levels of c-Jun and GFP⁺ iPSC clone numbers with the change of time period in c-Jun^{TetOn} ESC+OKS system were investigated. FIG. 2D shows changes in expression levels of c-Jun and GFP⁺ iPS clone numbers with the increase of the days using DOX. It can be seen that, without the addition of DOX, GFP⁺ iPS clones had the biggest number; with the increase of the days using DOX, expression levels of c-Jun enhanced gradually, while GFP⁺ iPS clone numbers decreased gradually, which suggests that c-Jun inhibited the reprogramming through the whole reprogramming process.

By studying the different roles that c-Jun plays in somatic cells and stem cells and the influences of c-Jun on the reprogramming of somatic cells as described above, the inventors of the present invention reached a conclusion that c-Jun can significantly inhibit pluripotent relative genes, thereby inhibiting the reprogramming of somatic cells and leading to the differentiation of stem cells. Therefore, the inventors of the present invention predict that the inhibitory effect of c-Jun on somatic cell reprogramming can be eliminated by antagonizing c-Jun activity, so that a c-Jun antagonist can be used to promote the formation of induced pluripotent stem cells.

### Example 2. Roles of a bZIP domain in c-Jun

### 1. Influences of various domains in c-Jun on the reprogramming of somatic cells

With molecular cloning techniques, the JNK-Ser63/Ser73 site phosphorylation mutants of c-Jun and the truncated c-Jun (c-JunDN) located between 170-334 amino acids and having a bZIP domain but lacking a transactivation domain were constructed. The wild-type c-JunWT had an amino acid sequence of SEQ ID No.1; and c-JunDN had an amino acid sequence of SEQ ID NO.2.

The amino acid sequence (SEQ ID NO.1) of the wild-type c-JunWT (i.e., the full-length c-Jun) :

The amino acid sequence (SEQ ID NO.2) of c-JunDN:

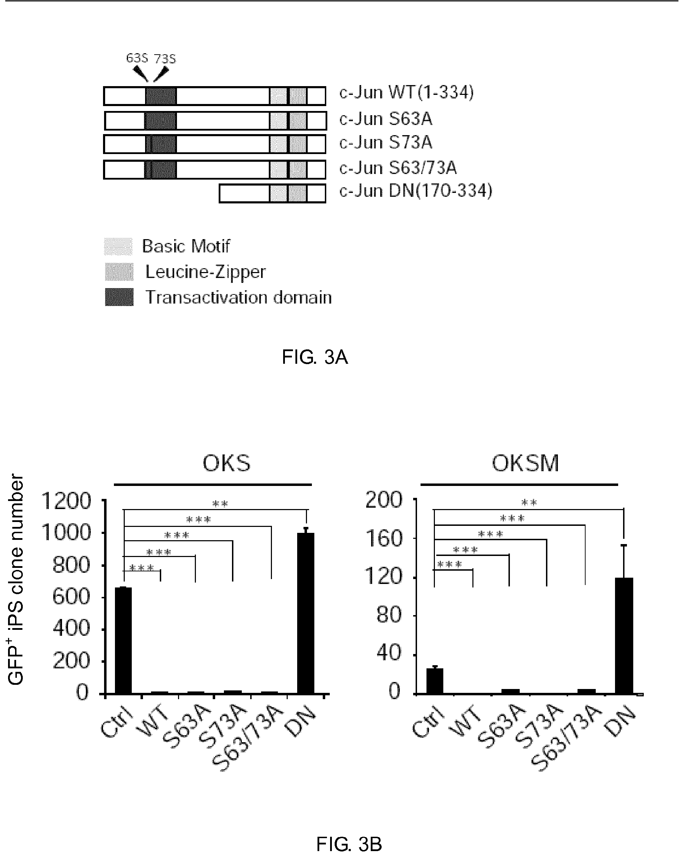

FIG. 3A shows schematic diagrams of protein sequences of the wild-type c-Jun (c-JunWT), the Ser63 site phosphorylation mutants (c-Jun S63A) and the Ser73 site phosphorylation mutants (c-Jun S73A), the Ser63 and Ser73 site phosphorylation mutants (c-Jun S63A/S73A) and the truncated c-Jun (c-JunDN) located between 170-334 amino acids and having a bZIP domain but lacking a transactivation domain.

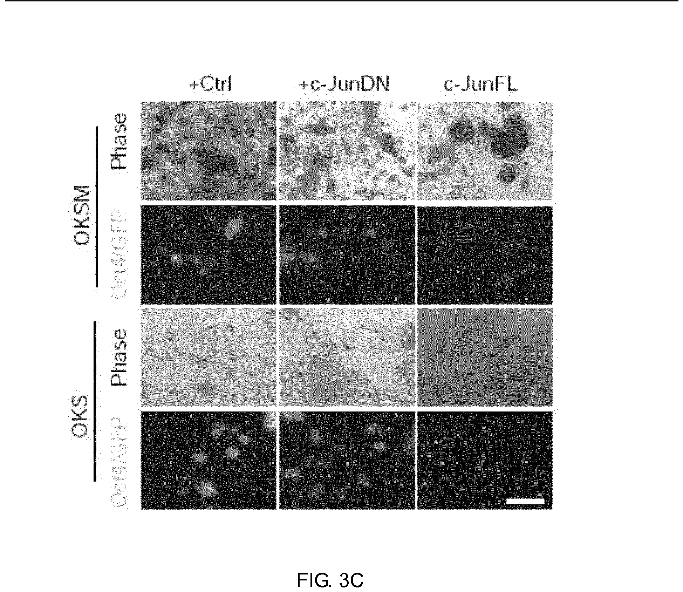

Then, according to the method for culturing cells and the method for detecting reprogramming efficiency as described in Example 1, the influences of adding c-JunWT, c-Jun S63A, c-Jun S73A, c-Jun S63A/S73A and c-JunDN to OKS and OKSM systems on the reprogramming efficiency were examined. FIG. 3B shows changes in clone number of GFP⁺ iPS after adding c-JunWT, c-Jun S63A, c-Jun S73A, c-Jun S63A/S73A and c-JunDN to OKS system and OKSM system, respectively, wherein the controls are OKS and OKSM systems themselves without the addition of any other factors. As shown in FIG. 3B, in OKS and OKSM systems with JNK-site phosphorylation mutants (i.e., c-Jun S63A, c-Jun S73A, c-Jun S63A/S73A) and c-JunWT added, GFP⁺ iPS clone number was almost zero, and in OKS and OKSM systems with c-Jun added, GFP⁺ iPS clone number increased significantly, suggesting that JNK-site phosphorylation mutants (i.e., c-Jun S63A, c-Jun S73A, c-Jun S63A/S73A) and c-JunWT had the same effect and completely blocked the reprogramming, while c-JunDN can significantly promote the reprogramming of somatic cells in OKS and OKSM systems. The stem cell fluorescence micrographs shown in FIG. 3C further confirmed this result. As shown in FIG. 3C, more stem cells were produced in OKS and OKSM systems with c-JunDN added, while nearly no stem cells were produced in OKS and OKSM systems with the wild-type c-Jun (c-JunWT) added, demonstrating that c-JunDN promoted the reprogramming of somatic cells to a large extent, thereby promoting the formation of induced pluripotent stem cells, while c-JunWT inhibited the reprogramming of somatic cells.

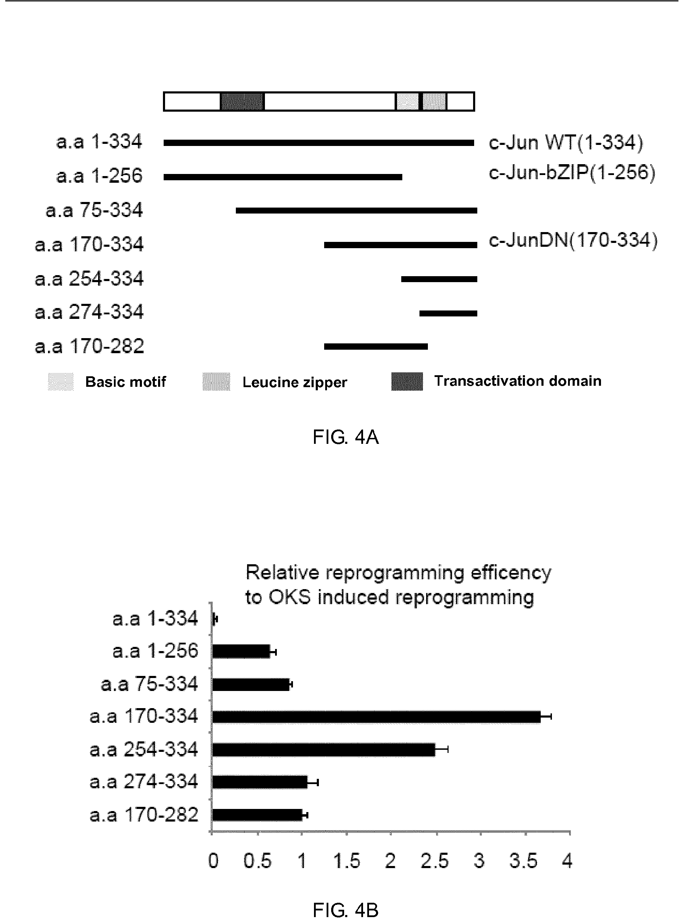

To further demonstrate the roles of various domains in c-Jun in the reprogramming of somatic cells, other truncated mutants of c-Jun except c-JunDN were constructed by the method as described in this example: the truncated c-Jun factors (c-Jun a.a 254-334) located between 254-334 amino acids, the truncated c-Jun factors (c-Jun a.a 274-334) located between 274-334 amino acids, the truncated c-Jun factors (c-Jun a.a 170-282) located between 170-282 amino acids, the c-Jun factors (c-Jun-bZIP, a.a 1-256) lacking a bZIP domain, and the c-Jun factors (c-Jun a.a 75-334) including a transactivation domain and a bZIP domain. FIG. 4A shows schematic diagrams of protein sequences of c-JunWT, c-Jun-bZIP, c-Jun a.a 75-334, c-JunDN, c-Jun a.a 254-334, c-Jun a.a 274-334 and c-Jun a.a 170-282.

Then, according to the method for culturing cells and the method for detecting reprogramming efficiency as described in Example 1, the influences of adding c-JunWT, c-Jun-bZIP, c-Jun a.a 75-334, c-JunDN, c-Jun a.a 254-334, c-Jun a.a 274-334 and c-Jun a.a 170-282 to OKS system on the reprogramming efficiency were examined. FIG. 4B shows the proportion of green fluorescent cells obtained by analysis using a flow cytometer after introducing the above-mentioned c-Jun factor mutants into OKS system. As shown in FIG. 4B, in OKS system with c-JunDN added, the proportion of green fluorescent cells was the highest, which was up to 3.5; in OKS system with c-Jun a.a 254-334 added, the proportion of green fluorescent cells was relatively low; in OKS system with c-JunWT added, the proportion of green fluorescent cells was the lowest (almost zero); in OKS system with c-Jun-bZIP and c-Jun a.a 75-334 added, the proportion of green fluorescent cells was less than 1; and in OKS system with c-Jun a.a 254-334, c-Jun a.a 274-334 and c-Jun a.a 170-282 added, the proportion of green fluorescent cells was slightly higher than 1. This result indicates that the truncated c-Jun factor having a bZIP domain but lacking a transactivation domain can significantly promote the reprogramming with a high efficiency, thereby promoting the formation of induced pluripotent stem cells. However, c-JunWT and c-Jun factors (c-Jun-bZIP) lacking a bZIP domain may inhibit the reprogramming, thereby hindering the formation of induced pluripotent stem cells.

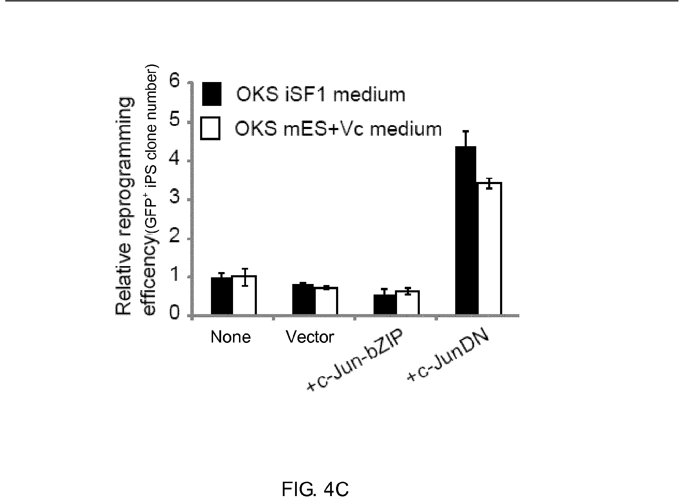

In order to further verify the influence of bZIP domain on the reprogramming, the effects of c-JunDN and c-Jun-bZIP on the reprogramming efficiency were compared. FIG. 4C shows clone number of GFP⁺ iPS obtained in iSF1 culture medium (high glucose culture medium DMEM, 10%KOSR, 0.5%N2, 1000U/mL LIF, 5ng/ml bFGF, 2mM L-glutamine, 1/100NEAA MEM, 0.1 mM mercaptoethanol, penicillin-streptomycin, please see Chinese Patent Application No. 200910038883.4 for the components contained in the culture medium and the amounts thereof) or mES culture medium supplemented with vitamin C, in OKS system with the addition of c-JunDN and c-Jun-bZIP respectively, wherein the control is OKS system itself without the addition of any other factors and the vector is pMXs. It can be seen from Fig. 4C that, in OKS system with the addition of c-JunDN, clone number of GFP⁺ iPS obtained in both iSF1 culture medium and mES culture medium supplemented with vitamin C increased significantly, while in OKS system with the addition of c-Jun-bZIP, clone number of GFP⁺ iPS obtained in each of the two kinds of culture media were less than clone number of GFP⁺ iPS generated in OKS system itself. This result shows that, the c-Jun-bZIP factor lacking a bZIP domain failed to promote the reprogramming, and therefore the bZIP domain was necessary for the reprogramming.

At the same time, the Jun dimer protein 2 (Jdp2) was isolated from the proteins of AP-1 family and its amino acid sequence was as follows:
The amino acid sequence (SEQ ID NO.3) of Jdp2:

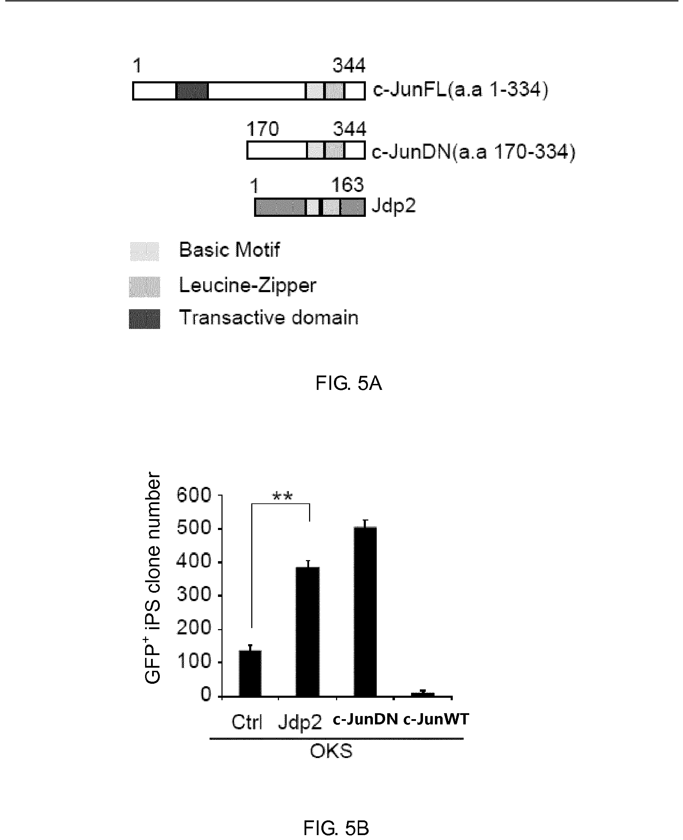

By sequence analysis, it was found that Jdp2 and c-JunDN had the same sequence structure. FIG. 5A shows schematic diagrams of the sequence structures of the full-length c-Jun (c-JunFL), c-JunDN and Jdp2. It can be seen from FIG. 5A that Jdp2 and c-JunDN had the same sequence structure, i.e., having a bZIP domain but lacking a transactivation domain. Subsequently, clone number of GFP⁺ iPS obtained in OKS system with the addition of c-JunFL, c-JunDN and Jdp2 were examined. FIG. 5B shows clone number of GFP⁺ iPS obtained after adding c-JunFL, c-JunDN and Jdp2 to OKS system, wherein the control is OKS system itself. As shown in FIG. 5B, compared with the c-JunFL+OKS system, similarly, both Jdp2 and c-JunDN can significantly promote somatic cell reprogramming in OKS system.

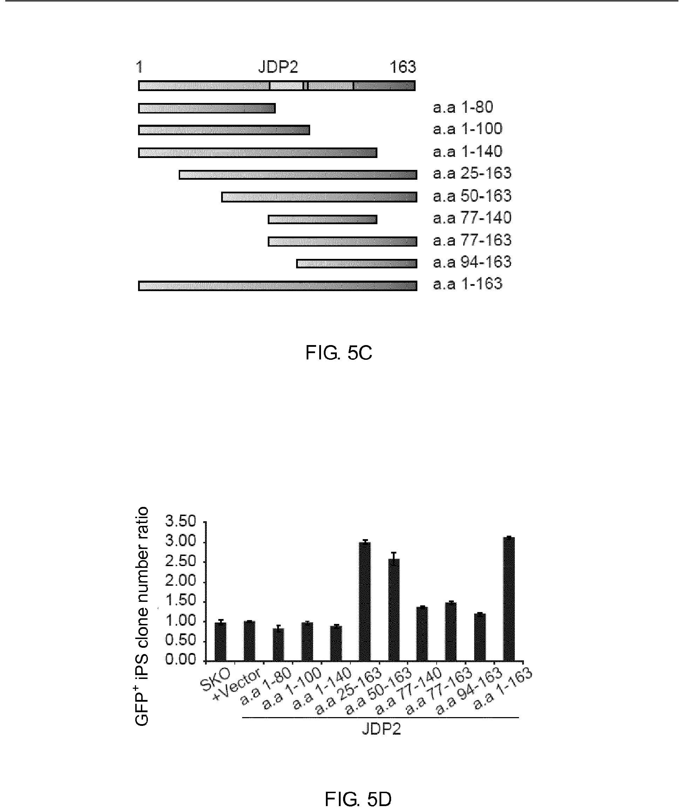

The invention also constructed various different mutants of Jdp2, whose protein sequences were schematically shown in FIG. 5C. These mutants include mutants (a.a.1-80 and a.a.1-100) having different lengths and lacking a bZIP domain and mutants (a.a.1-140, a.a.25-163, a.a.50-163, a.a.77-140, a.a.77-163, a.a.94-163) having a bZIP domain. Subsequently the effects of adding these Jdp2 mutants to OKS system on the reprogramming efficiency were studied. FIG. 5D shows the proportion of green fluorescent cells obtained by analysis using a flow cytometer after introducing the above-mentioned Jdp2 mutants into OKS system. It can be seen from FIG. 5D that the full-length Jdp2 had the highest proportion of green fluorescent cells (higher than 3.0), Jdp2 mutants a.a.25-163 having a bZIP domain had a relatively higher proportion of green fluorescent cells, and Jdp2 mutants (a.a.1-80 and a.a.1-100) lacking a bZIP domain had the proportion of green fluorescent cells of less than 1.0, which further demonstrates that the bZIP domain was necessary for promoting the reprogramming.

### Example 3. Identification of the obtained induced pluripotent stem cells

Taking c-JunDN/Jdp2+KSM (Klf4, Sox2 and c-Myc) and c-JunDN/Jdp2+KS (Klf4 and Sox2) systems for instance, according to the method and culture medium as described in the above Example 1, mouse embryonic fibroblasts were used to culture induced pluripotent stem cells (iPSCs) and the obtained induced pluripotent stem cells were analyzed and identified as follows.

### i. PCR Analysis-Exogenous Gene Integration Analysis

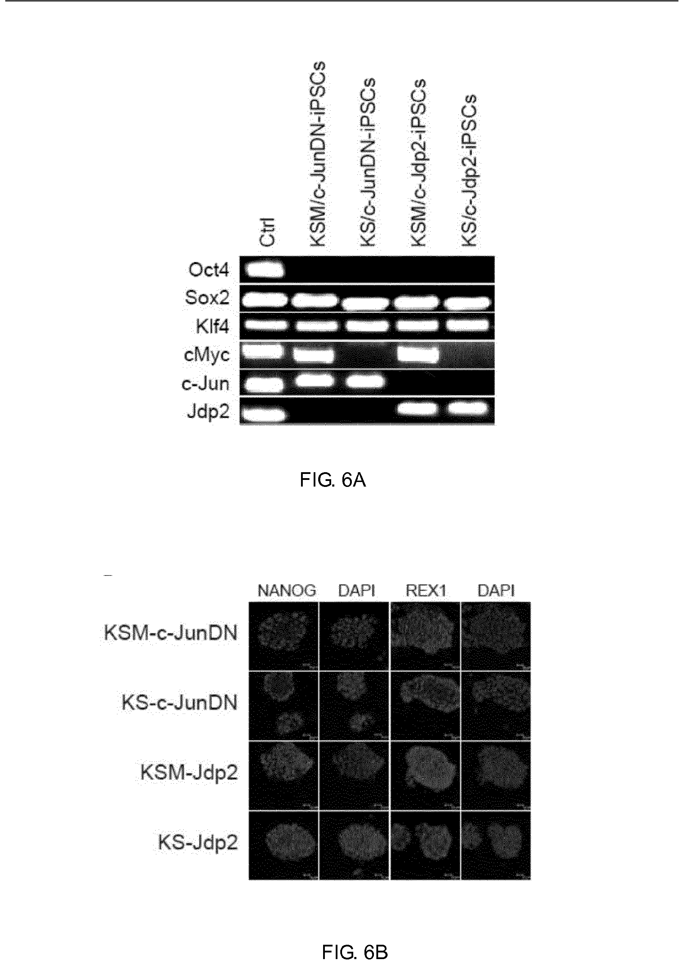

As shown in FIG. 6A, exogenous factors were detected in cells, indicating the integration of exogenous factors in iPSCs.

### ii. Analysis of Methylation Status of Promoter Region

Analysis of methylation status of promoter regions was carried out by sodium bisulfite sequencing well-known in the art.

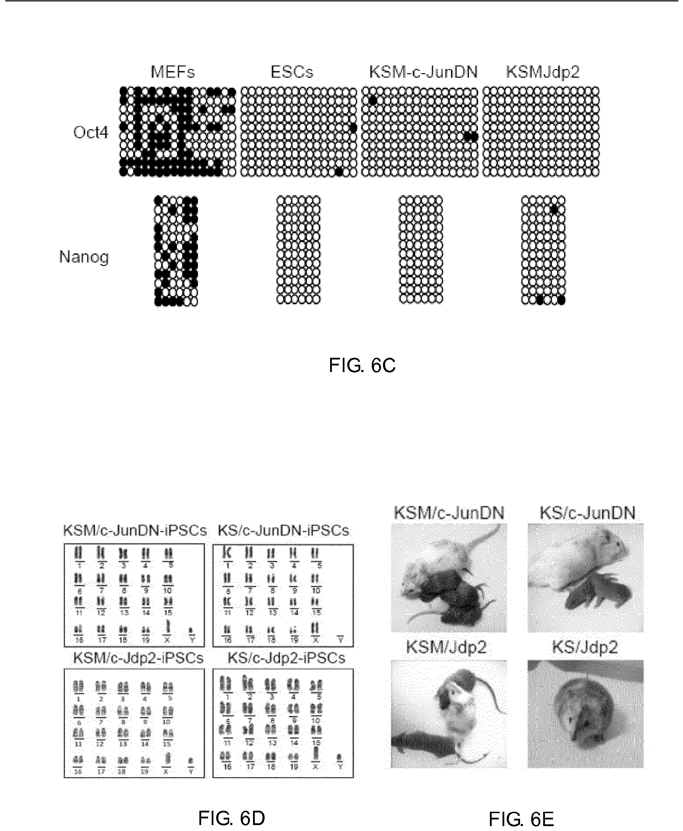

FIG. 6B shows immune fluorescence images of induced pluripotent stem cells generated in c-JunDN/Jdp2+KSM system and c-JunDN/Jdp2+KS system, and it is demonstrated that the induced pluripotent stem cells obtained in the two systems expressed not only REX1 but also Nanog. FIG. 6C shows analysis results of methylation status of CpG islands located in Oct4 and Nanog promoter regions of induced pluripoent stem cells obtained in c-JunDN/Jdp2+KSM system, wherein the black parts indicate methylation and the white parts indicate absence of methylation. It can be seen from FIG. 6C that CpG islands located in Oct4 and Nanog promoter regions of induced pluripotent stem cells obtained in c-JunDN/Jdp2+KSM system had a similar demethylation status to mouse embryonic stem cells. Nanog and Oct4 are genes that are specifically expressed by embryonic stem cells and their expression states are closely related to the cell fate. These results demonstrate that this cells obtained by using c-JunDN/Jdp2+KSM system had changed fate, that is to say, they were induced into pluripotent stem cells.

### iii. Identification of Karyotypes of iPSCs

Next, the identification of karyotypes of iPSCs was conducted according to the method as described below.

The identification of karyotypes of iPSCs was carried out according to the following method:

Under feeder-free culture condition, iPS clones were cultured. The culture medium was replaced with a freshly prepared medium when cells grew to the cell density of 80% - 90% and colchicine with a final concentration of 0.2µg/ml was added. The resulting mixture was placed in an incubator at 37°C for 60 minutes. The culture medium was drawn off. The cells were washed with PBS, digested with 0.25% trypsin and collected. Under 250g condition, the cells were centrifuged for 5 minutes and then 7 ml of a KCl solution (0.075 mol/L, preheated at 37°C) was added. The cells were blown homogeneously and placed in a waterbath at 37°C for 20 minutes.

The cells were pre-fixed with a freshly prepared Carnoy's fixative for 3 minutes. After pre-fixation, the resultant mixture was centrifuged at 1200rpm/min for 5 minutes. The supernatant was discarded, about 7ml of a freshly prepared fixative was added thereto and the resultant mixture was placed in a waterbath at 37°C for 40 minutes for fixation. Centrifugal treatment was carried out at 1200rpm/min for 5 minutes. A large part of fixative was discarded and the left part was used to re-suspend cells. The resulting cell suspension was dropped onto frozen clean slides which were then placed in a drying oven at 75°C for 3 hours immediately.

0.03g trypsin powder was dissolved in 55 ml of saline water to produce a trypsin solution and the trypsin solution was adjusted to a pH of 7.2 with 3 % Tris. Subsequently, the prepared slides were put into the trypsin solution for 8 seconds and then placed into saline water quickly to terminate digestion. The resulting slides were placed in Giemsa staining solution to stain the cells for 5 to 10 minutes and then taken out with a forceps and rinsed with tap water gently. After drying at room temperature or with a hair dryer, mitosis was observed under oil microscope.

Results of karyotype experiment were shown in FIG. 6D. It was shown that induced pluripotent stem cells cultured in c-JunDN/Jdp2+KSM system and c-JunDN/Jdp2+KS system had normal karyotypes, demonstrating that the generated induced pluripotent stem cells had good quality and no abnormal chromosome was produced.

### iv. Blastula chimera test

Subsequently, iPSCs were injected into the blastocoele of donor mice, and then the injected blastulas were transplanted into the uteruses of pseudo-pregnant female mice to produce chimeric mice. Whether the born mice produce chimera was determined based on their coat color. FIG. 6E shows chimeric mice obtained by using induced pluripotent stem cells generated in c-JunDN/Jdp2+KSM system and c-JunDN/Jdp2+KS system according to the present invention, wherein the generated mice of the first generation had multicolored coat color, while the generated mice of the second generation had pure coat color, suggesting the generation of chimeric mice with germline transmission.

### Example 4. c-JunDN/Jdp2 can replace Oct4 to promote somatic cell reprogramming

### i. c-JunDN and Jdp2 regulate the same gene groups as Oct4

c-Jun, c-JunDN, and Jdp2 were overexpressed in mouse embryonic fibroblasts, and the gene groups regulated by them were detected by the following RNA-seq method.

RNA-seq method: firstly, total RNAs were prepared using TRIzol. Then, TruSeq RNA Sample Prep Kit (RS-122-2001, Illumina) was used for library constructions and sequencing done with Miseq Reagent Kit V2 (MS-102-2001) for RNA-seq.

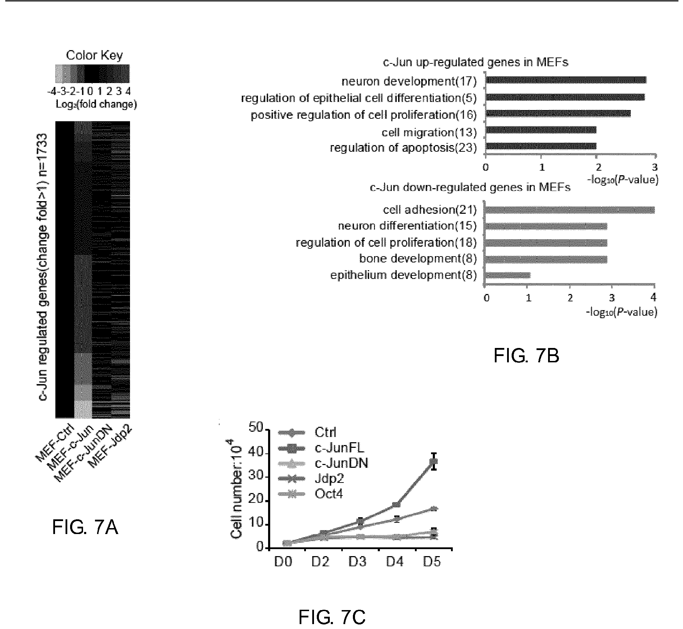

As shown in FIGS. 7A and 7B, 1733 genes regulated by c-Jun mainly regulated neural development, cell proliferation and differentiation, migration and apoptosis, and cell adhesion. FIG. 7C shows influences of c-Jun, Oct4, c-JunDN and Jdp2 on cell number of mouse embryonic fibroblasts with the increase of culture days, wherein the control is the growth of mouse embryonic fibroblasts infected with an empty vector. It can be seen that, c-JunDN and Jdp2 can inhibit the proliferation of mouse embryonic fibroblasts in a way similar to Oct4, while c-Jun obviously promoted the proliferation of mouse embryonic fibroblasts, which manifests that c-Jun regulated different genes from c-JunDN and Jdp2, while c-Jun regulated substantially the same genes as c-JunDN and Oct4 did.

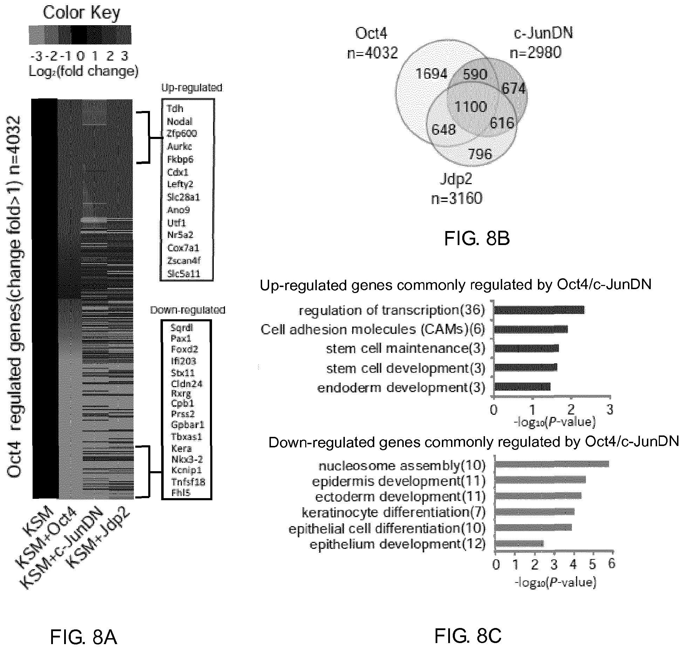

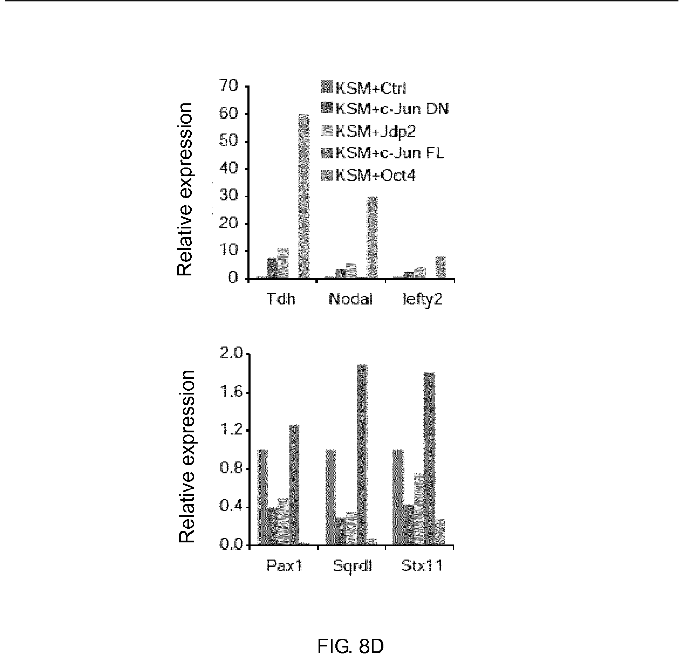

Next, gene regulation during mouse embryonic fibroblast reprogramming commonly induced by c-JunDN, Jdp2 or Oct4 in combination with KSM (Klf4, Sox2 and c-Myc) was studied. Mouse embryonic fibroblasts were cultured in c-JunDN+KSM, Jdp2 +KSM and Oct4+KSM systems respectively, according to the method and the condition for culturing somatic cells as described in Example 1. During transforming mouse embryonic fibroblasts into induced pluripotent cells, gene groups regulated by c-JunDN, Jdp2 and Oct4 were detected by the above-mentioned RNA-seq method. As shown in FIGS. 8A to 8C, gene groups regulated by these three factors were largely similar to each other in that they co-regulated 1100 genes including those that played important roles in pluripotency maintenance, nucleosome assembly, and epithelial cells differentiation, for example, Tdh, Nodal, Lefhy2, Pax1, Sqrd1, Stx11, and so on. Taking Tdh, Nodal, Lefhy2, Pax1, Sqrd1 and Stx11 for instance, qRT-PCR analysis was conducted and results were shown in FIG. 8D. These representative genes were co-regulated by c-JunDN, Jdp2 and Oct4. It is demonstrated that c-JunDN or Jdp2 can replace Oct4 to induce somatic cell reprogramming.

### ii. c-JunDN or Jpd2 can replace Oct4 to promote somatic cell reprogramming

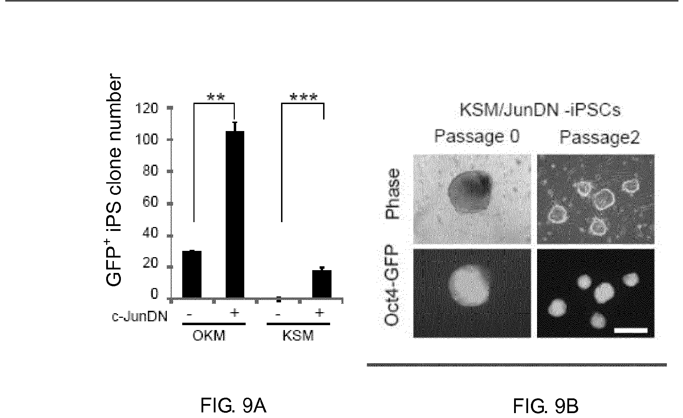

According to the culture method and the culture conditions as described in Example 1, cells were cultured in KSM+c-JunDN and OKM (i.e., Oct4, Klf4 and c-Myc) +c-JunDN systems and clone number of GFP⁺ iPS were detected, to determine the influences of the two systems on the efficiency of reprogramming. FIG. 9A shows clone number of GFP⁺ iPS obtained in KSM+c-JunDN and OKM+c-JunDN systems, wherein, "-" indicates no c-JunDN was added and "+" indicates c-JunDN was added. As shown in FiG. 9A, clone number of GFP⁺ iPS obtained in OKM system with the addition of c-JunDN increased remarkably, compared with OKM system without the addition of c-JunDN, and, the addition of c-JunDN to KSM system without Oct4 caused clone number of GFP⁺ iPS to increase remarkably, compared with KSM system without the addition of c-JunDN. These results demonstrate that c-JunDN can replace Oct4 to improve the reprogramming efficiency in KSM system, thereby promoting the generation of iPSCs, and c-JunDN can also replace Sox2 to improve the reprogramming efficiency in OKM system, thereby promoting the generation of iPSCs.

Furthermore, GFP⁺ iPS clones obtained in KSM+c-JunDN system were observed by fluorescence microscopy imaging, as shown in FIG. 9B. It can be seen that iPSC numbers in KSM system with the addition of c-JunDN (passage 2) increased significantly, compared with KSM system without the addition of c-JunDN (passage 0). This suggested that c-JunDN can replace Oct4 to improve the reprogramming efficiency in KSM system, thereby promoting the generation of iPSCs.

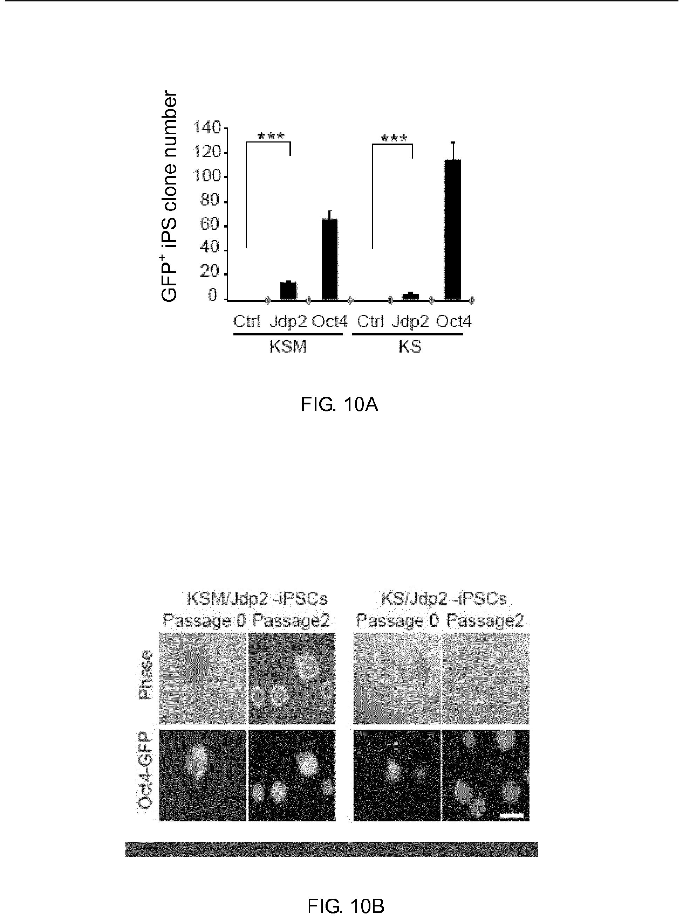

Similarly, according to the culture method and the culture conditions as described in Example 1, cells were cultured in KSM+Jdp2 and KS+Jdp2 systems and clone number of GFP⁺ iPS were detected, to determine the influences of the two systems on the efficiency of reprogramming. FIG. 10A shows clone number of GFP⁺ iPS obtained in KSM+Jdp2 and KS+Jdp2 systems, wherein, the control is KSM or KS system without the addition of Jdp2. As shown in FiG. 10A, clone number of GFP⁺ iPS obtained both in KSM system with the addition of Jdp2 and in KS system with the addition of Jdp2 increased remarkably, compared with KSM or KS system without the addition of c-JunDN. These results demonstrate that Jdp2 can replace Oct4 to improve the reprogramming efficiency in KSM system and KS system, thereby promoting the generation of iPSCs.

Similarly, GFP⁺ iPS clones obtained in KSM+Jdp2 system and KS+Jdp2 system were observed by fluorescence microscopy imaging, as shown in FIG. 10B. iPSC numbers in KSM system and KS system with the addition of Jdp2 (passage 2) increased significantly, compared with KSM system and KS system without the addition of Jdp2 (passage 0). This suggests that Jdp2 can replace Oct4 to improve the reprogramming efficiency in KSM system and KS system, thereby promoting the generation of iPSCs.

### Example 5. Six-factor system that can promote the reprogramming of somatic cells

Previous research had identified the factor Jhdmlb that can promote Oct4 single factor reprogramming [Wang et al., 2011], the BMP downstream factor Id1/3 [Chen et al., 2011b] and the three factors Glis1, Sall4 and Lrhl that can enhance the efficiency of reprogramming [Buganim et al., 2012; Heng et al., 2010; Maekawa et al., 2011].

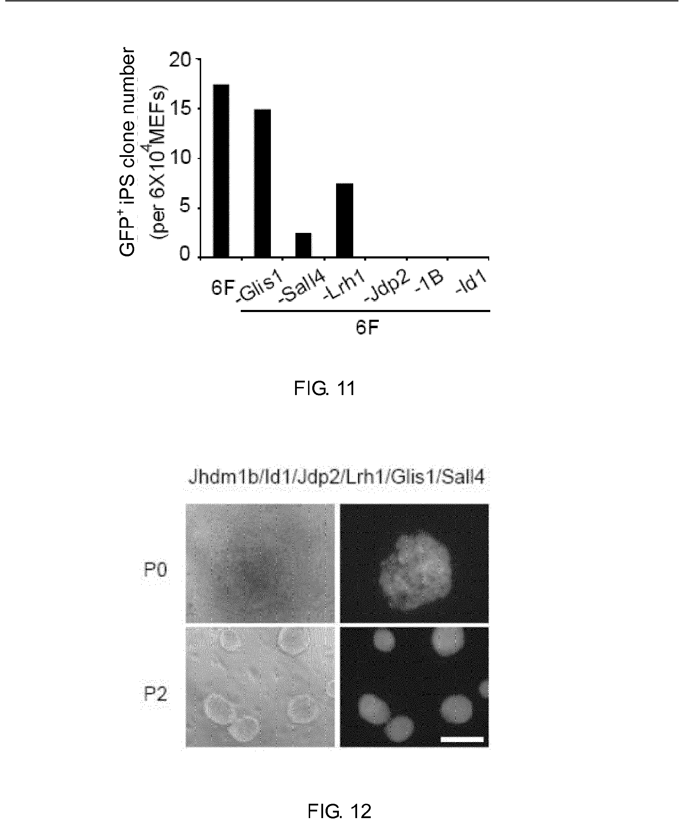

The inventors of the present invention combined c-JunDN/Jdp2 with Jhdm1b, Id1/3, Glis1, Sall4, and Lrhl to form a six-factor (6F) system based on the above experimental results: c-JunDN/Jdp2 can replace Oct4 to promote the reprogramming in KSM or KS system, thereby promoting the formation of induced pluripotent stem cells, and cultured mouse embryonic fibroblasts under the six-factor condition according to the culture method and the culture conditions as described in Example 1, to detect clone number of GFP⁺ iPS of the obtained cells. As shown in FIG. 11, clone number of GFP⁺ iPS of the obtained cells cultured in the 6F system were up to about 18, which shows that mouse embryonic fibroblasts had been successfully induced into pluripotent stem cells., Then, any one factor was eliminated from the six factors to form the following five-factor systems:
(1) c-JunDN/Jdp2+Jhdm1b+Id1/3+G1is1+Sall4,
(2) c-JunDN/Jdp2+Jhdm1b+Id1/3+Glis1+Lrh1,
(3) c-JunDN/Jdp2+Jhdm1b+Id1/3+Lrh1+Sall4,
(4) c-JunDN/Jdp2+Jhdm1b+Glis1+Sall4+Lrh1,
(5) Jhdm1b+Id1/3+Glis1+Sall4+Lrh1, and
(6) c-JunDN/Jdp2+Id1/3+Glis1+Sall4+Lrh1.

Similarly, according to the culture method and the culture conditions as described in Example 1, mouse embryonic fibroblasts were cultured under the above five-factor conditions respectively and clone number of GFP⁺ iPS of the obtained cells were detected. As shown in FIG. 11, in a five-factor system absent of any one of Glis1, Sall4 and Lrh1, clone number of GFP⁺ iPS were also relatively large; while in a five-factor system absent of any one of c-JunDN/Jdp2, Jhdmlb and Id1/3, clone number of GFP⁺ iPS were almost zero. It was demonstrated that c-JunDN/Jdp2, Jhdmlb or Id1/3 were necessary for somatic cell reprogramming, and a five-factor system absent of any one of Glis1, Sall4 and Lrhl could also promote somatic cell reprogramming, thereby promoting the formation of induced pluripotent stem cells.

The influence of the 6F system on the formation of induced pluripotent stem cells was observed by cell fluorescence microscopy imaging. As shown in the cell fluorescence micrographs of FIG. 12, in the 6F system pluripotent stem cells significantly increased.

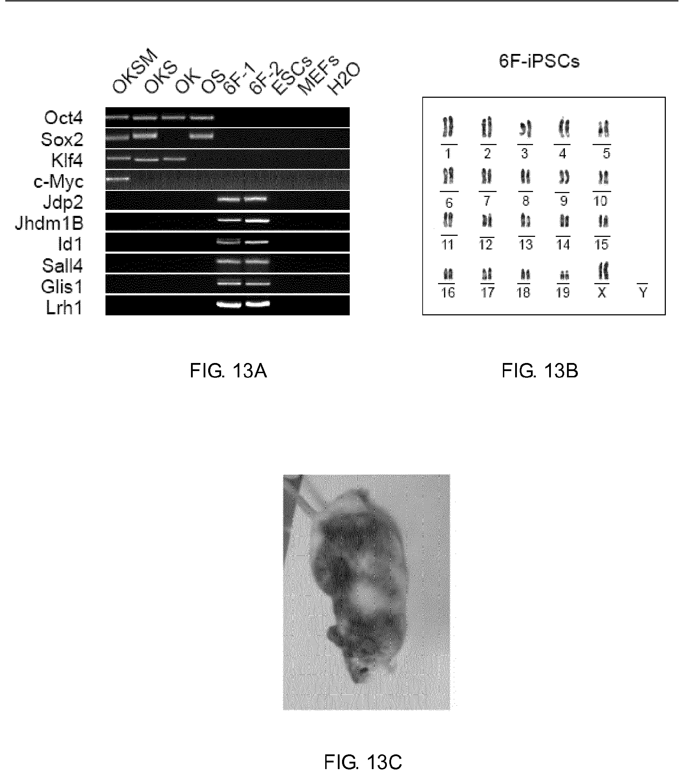

Induced pluripotent stem cells obtained in the 6F system were analyzed and identified according to the PCR method, the method for identifying karyotypes and the method for testing blastula chimera as described in Example 3 and the results were shown in FIGS. 13A to 13C. FIG. 13A shows the results of PCR analysis and it is shown that the obtained iPSCs did not integrate Yamanaka factors. FIG. 13B shows karyotype experimental results and it is shown that the induced pluripotent stem cells cultured in the 6F system had normal karyotypes. This suggests that the generated induced pluripotent stem cells had good quality and no abnormal chromosome was produced. FIG. 13C shows a photograph of the chimeric mouse obtained by injecting iPSCs generated in the 6F system into the blastocoele of a donor mouse and then transplanting the injected blastula into the uterus of a pseudo-pregnant female mouse. As can be seen from FIG. 13C, the multicolored coat color of the mouse demonstrates that the induced pluripotent stem cells cultured in the 6F system can generate a chimeric mouse.

Although the preferred embodiments of the present invention have been described and illustrated herein, it is obvious to those skilled in the art that these embodiments are only illustrative. It will be apparent to those skilled in the art that numerous variations, modifications and substitutions can be made to these embodiments. The scope of the present invention is defined by the appended claims and the methods and structures as fall within the claims together with the equivalents thereof are intended to be embraced by the appended claims.
## Claims

1. A method for preparing an induced pluripotent stem cell, comprising introducing a composition for promoting the formation of an induced pluripotent stem cell into a mammalian somatic cell,
wherein, said composition comprises nucleic acids encoding the following polypeptides: a c-Jun antagonist, Jhdm1b, Id1, and at least one factor selected only from Glis1, Sall4 and Lrh1;
wherein the c-Jun antagonist comprises:
c-JunDN having the amino acid sequence of SEQ ID NO. 2 or a functional variant thereof, wherein the functional variant thereof has a bZIP domain but lacks a transactivation domain and shows at least 70% homology to SEQ ID NO. 2, or
Jdp2 having the amino acid sequence of SEQ ID NO. 3 or a functional variant thereof, wherein the functional variant thereof has a bZIP domain but lacks a transactivation domain and shows at least 70% homology to SEQ ID NO. 3; and
wherein, when the c-Jun antagonist is c-JunDN, said composition does not comprise nucleic acids encoding the following four groups of polypeptides: (1) Sox2, Klf4 and c-Myc, (2) Oct3/4, Klf4 and c-Myc, (3) Oct3/4, Lkf and Sox2, or (4) Klf4 and Sox2.

2. The method of claim 1, wherein the nucleic acids encode:
i) a c-Jun antagonist, Jhdm1b, Id1, Glis1, Sall4, and Lrh1;
ii) a c-Jun antagonist, Jhdm1b, Id1, Glis1, and Sall4;
iii) a c-Jun antagonist, Jhdm1b, Id1, Glis1, and Lrh1; or
iv) a c-Jun antagonist, Jhdm1b, Id1, Sall4, and Lrh1.

3. The method of claim 1 or 2, wherein the composition comprises one or more expression vectors comprising the nucleic acids encoding the polypeptides.

4. The method of claim 3, wherein the one or more expression vectors are selected from the group consisting of plasmids, cosmids, viral vectors, and artificial chromosomes.

5. The method of claim 4, wherein the viral vectors are selected from the group consisting of retroviral vectors, lentiviral vectors, adenoviral vectors, adeno-associated viral vectors, simian virus 40 vectors, bovine papilloma virus vectors, Epstein-Barr virus, herpes virus vectors, vaccinia virus vectors, Harvey murine sarcoma virus vectors, murine mammary tumor virus vectors, and Rous sarcoma virus vectors.

6. The method of any one of claims 1 to 5, wherein, the mammalian somatic cell is selected from mouse somatic cell or human somatic cell.

7. The method of claim 6, wherein, the mouse somatic cell or the human somatic cell are selected from the group consisting of: fibroblast, bone marrow derived mononuclear cell, skeletal muscle cell, fat cell, peripheral blood mononuclear cell, macrophage, neural stem cell, hepatic cell, keratinocyte, oral keratinocyte, hair follicle dermal cell, gastric epithelial cell, lung epithelial cell, synovial cell, renal cell, skin epithelial cell and osteoblast cell.

8. A composition for promoting the formation of an induced pluripotent stem cell,
wherein, said composition comprises nucleic acids encoding the following polypeptides: a c-Jun antagonist, Jhdm1b, Id1, and at least one factor selected only from Glis1, Sall4 and Lrh1;
wherein the c-Jun antagonist comprises:
c-JunDN having the amino acid sequence of SEQ ID NO. 2 or a functional variant thereof, wherein the functional variant thereof has a bZIP domain but lacks a transactivation domain and shows at least 70% homology to SEQ ID NO. 2, or
Jdp2 having the amino acid sequence of SEQ ID NO. 3 or a functional variant thereof, wherein the functional variant thereof has a bZIP domain but lacks a transactivation domain and shows at least 70% homology to SEQ ID NO. 3, and
wherein, when the c-Jun antagonist is c-JunDN, said composition does not comprise nucleic acids encoding the following four groups of polypeptides: (1) Sox2, Klf4 and c-Myc, (2) Oct3/4, Klf4 and c-Myc, (3) Oct3/4, Lkf and Sox2, or (4) Klf4 and Sox2.

9. The composition of claim 8, wherein the nucleic acids encode:
i) a c-Jun antagonist, Jhdm1b, Id1, Glis1, Sall4, and Lrh1;
ii) a c-Jun antagonist, Jhdm1b, Id1, Glis1, and Sall4;
iii) a c-Jun antagonist, Jhdm1b, Id1, Glis1, and Lrh1; or
iv) a c-Jun antagonist, Jhdm1b, Id1, Sall4, and Lrh1.

10. The composition of claim 8 or 9, wherein the composition comprises one or more expression vectors comprising the genes encoding the polypeptides.

11. The composition of claim 10, wherein the one or more expression vectors are selected from the group consisting of plasmids, cosmids, viral vectors, and artificial chromosomes.

12. The composition of claim 11, wherein the viral vectors are selected from the group consisting of retroviral vectors, lentiviral vectors, adenoviral vectors, adeno-associated viral vectors, simian virus 40 vectors, bovine papilloma virus vectors, Epstein-Barr virus, herpes virus vectors, vaccinia virus vectors, Harvey murine sarcoma virus vectors, murine mammary tumor virus vectors, and Rous sarcoma virus vectors.

13. Use of the composition of any one of claims 8 to 12 in the preparation of an induced pluripotent stem cell.

## Patentansprüche

1. Verfahren zur Herstellung einer induzierten pluripotenten Stammzelle, umfassend das Einführen einer Zusammensetzung zur Förderung der Bildung einer induzierten pluripotenten Stammzelle in eine somatische Säugerzelle,
wobei die Zusammensetzung Nukleinsäuren umfasst, die für die folgenden Polypeptide codieren: einen c-Jun-Antagonisten, Jhdm1b, Id1 und wenigstens einen Faktor, ausgewählt nur aus Glis1, Sall4 und Lrh1;
wobei der c-Jun-Antagonist umfasst:
c-JunDN mit der Aminosäuresequenz von SEQ ID Nr. 2 oder einer funktionellen Variante davon, wobei die funktionelle Variante davon eine bZIP-Domäne aufweist, aber keine Transaktivierungsdomäne aufweist und wenigstens 70 % Homologie zu SEQ ID Nr. 2 zeigt, oder
Jdp2 mit der Aminosäuresequenz von SEQ ID Nr. 3 oder einer funktionellen Variante davon, wobei die funktionelle Variante davon eine bZIP-Domäne aufweist, aber keine Transaktivierungsdomäne aufweist und wenigstens 70 % Homologie zu SEQ ID Nr. 3 zeigt; und
wobei, wenn der c-Jun-Antagonist c-JunDN ist, die Zusammensetzung keine Nukleinsäuren umfasst, die für die folgenden vier Gruppen von Polypeptiden codieren: (1) Sox2, Klf4 und c-Myc, (2) Oct3/4, Klf4 und c-Myc, (3) Oct3/4, Lkf und Sox2 oder (4) Klf4 und Sox2.

2. Verfahren nach Anspruch 1, wobei die Nukleinsäuren für Folgendes codieren:
i) einen c-Jun-Antagonisten, Jhdm1b, Id1, Glis1, Sall4 und Lrh1;
ii) einen c-Jun-Antagonisten, Jhdm1b, Id1, Glis1 und Sall4;
iii) einen c-Jun-Antagonisten, Jhdm1b, Id1, Glis1 und Lrh1; oder
iv) einen c-Jun-Antagonisten, Jhdm1b, Id1, Sall4 und Lrh1.

3. Verfahren nach Anspruch 1 oder 2, wobei die Zusammensetzung einen oder mehrere Expressionsvektoren umfasst, welche die Nukleinsäuren umfassen, welche für die Polypepdide codieren.

4. Verfahren nach Anspruch 3, wobei der eine oder die mehreren Expressionsvektoren aus der Gruppe ausgewählt werden, die aus Plasmiden, Cosmiden, viralen Vektoren und künstlichen Chromosomen besteht.

5. Verfahren nach Anspruch 4, wobei die viralen Vektoren aus der Gruppe ausgewählt werden, die aus retroviralen Vektoren, lentiviralen Vektoren, adenoviralen Vektoren, adenoassoziierten viralen Vektoren, Simianvirus-40-Vektoren, Rinderpapillomavirusvektoren, Epstein-Barr-Virus, Herpesvirus-Vektoren, Vacciniavirusvektoren, Harvey Murine Sarcoma Virus-Vektoren, Maus-Mammatumorvirus-Vektoren und Rous-Sarkomvirus-Vektoren besteht.

6. Verfahren nach einem der Ansprüche 1 bis 5, wobei, die somatische Säugerzelle aus einer somatischen Zelle der Maus oder einer somatischen Zelle des Menschen ausgewählt wird.

7. Verfahren nach Anspruch 6, wobei, die somatische Zelle der Maus oder die somatische Zelle des Menschen aus der Gruppe ausgewählt werden, die aus einem Fibroblasten, einer von Knochenmark abgeleiteten mononukleären Zelle, einer Skelettmuskelzelle, einer Fettzelle, einer mononukleären Zelle des peripheren Bluts, einem Makrophagen, einer neuralen Stammzelle, einer Leberzelle, einem Keratinozyten, einem oralen Keratinozyten, einer Haarfollikel-Hautzelle, einer Magenepithelzelle, einer Lungenepithelzelle, einer Synoviazelle, einer Nierenzelle, einer Hautepithelzelle und einer Osteoblastenzelle besteht.

8. Zusammensetzung zur Förderung der Bildung einer induzierten pluripotenten Stammzelle,
wobei die Zusammensetzung Nukleinsäuren umfasst, die für die folgenden Polypeptide codieren: einen c-Jun-Antagonisten, Jhdm1b, Id1 und wenigstens einen Faktor, ausgewählt nur aus Glis1, Sall4 und Lrh1;
wobei der c-Jun-Antagonist umfasst:
c-JunDN mit der Aminosäuresequenz von SEQ ID Nr. 2 oder einer funktionellen Variante davon, wobei die funktionelle Variante davon eine bZIP-Domäne aufweist, aber keine Transaktivierungsdomäne aufweist und wenigstens 70 % Homologie zu SEQ ID Nr. 2 zeigt, oder
Jdp2 mit der Aminosäuresequenz von SEQ ID Nr. 3 oder einer funktionellen Variante davon, wobei die funktionelle Variante davon eine bZIP-Domäne aufweist, aber keine Transaktivierungsdomäne aufweist und wenigstens 70 % Homologie zu SEQ ID Nr. 3 zeigt, und
wobei, wenn der c-Jun-Antagonist c-JunDN ist, die Zusammensetzung keine Nukleinsäuren umfasst, die für die folgenden vier Gruppen von Polypeptiden codieren: (1) Sox2, Klf4 und c-Myc, (2) Oct3/4, Klf4 und c-Myc, (3) Oct3/4, Lkf und Sox2 oder (4) Klf4 und Sox2.

9. Zusammensetzung nach Anspruch 8, wobei die Nukleinsäuren für Folgendes codieren:
i) einen c-Jun-Antagonisten, Jhdmlb, Id1, Glis1, Sall4 und Lrh1;
ii) einen c-Jun-Antagonisten, Jhdm1b, Id1, Glis1 und Sall4;
iii) einen c-Jun-Antagonisten, Jhdmlb, Id1, Glis1 und Lrh1; oder
iv) einen c-Jun-Antagonisten, Jhdm1b, Id1, Sall4 und Lrh1.

10. Zusammensetzung nach Anspruch 8 oder 9, wobei die Zusammensetzung einen oder mehrere Expressionsvektoren umfasst, welche die Gene umfassen, welche für die Polypeptide codieren.

11. Zusammensetzung nach Anspruch 10, wobei der eine oder die mehreren Expressionsvektoren aus der Gruppe ausgewählt werden, die aus Plasmiden, Cosmiden, viralen Vektoren und künstlichen Chromosomen besteht.

12. Zusammensetzung nach Anspruch 11, wobei die viralen Vektoren aus der Gruppe ausgewählt werden, die aus retroviralen Vektoren, Antiviralen Vektoren, adenoviralen Vektoren, adenoassoziierten viralen Vektoren, Simianvirus-40-Vektoren, Rinderpapillomavirusvektoren, Epstein-Barr-Virus, Herpesvirus-Vektoren, Vacciniavirusvektoren, Harvey Murine Sarcoma Virus-Vektoren, Maus-Mammatumorvirus-Vektoren und Rous-Sarkomvirus-Vektoren besteht.

13. Verwendung der Zusammensetzung nach einem der Ansprüche 8 bis 12 bei der Herstellung einer induzierten pluripotenten Stammzelle.

## Revendications

1. Un procédé pour préparer une cellule souche pluripotente induite, comprenant l'introduction d'une composition pour favoriser la formation d'une cellule souche pluripotente induite dans une cellule somatique de mammifère,
dans lequel, ladite composition comprend des acides nucléiques codant pour les polypeptides suivants : un antagoniste c-Jun, Jhdm1b, Id1, et au moins un facteur choisi uniquement parmi Glis1, Sall4 et Lrh1 ;
dans lequel l'antagoniste c-Jun comprend :
c-JunDN ayant la séquence d'acides aminés de SEQ ID NO. 2 ou une variante fonctionnelle de celle-ci, dans lequel la variante fonctionnelle de celle-ci a un domaine bZIP mais manque d'un domaine de transactivation et présente au moins 70% d'homologie avec SEQ ID NO. 2, ou
Jdp2 ayant la séquence d'acides aminés de SEQ ID NO. 3 ou une variante fonctionnelle de celle-ci, dans lequel la variante fonctionnelle de celle-ci a un domaine bZIP mais manque d'un domaine de transactivation et présente au moins 70% d'homologie avec SEQ ID NO. 3 ; et
dans lequel, lorsque l'antagoniste c-Jun est c-JunDN, ladite composition ne comprend pas d'acides nucléiques codant pour les quatre groupes de polypeptides suivants : (1) Sox2, Klf4 et c-Myc, (2) Oct3/4, Klf4 et c-Myc, (3) Oct3/4, Lkf et Sox2, ou (4) Klf4 et Sox2.

2. Le procédé selon la revendication 1, dans lequel les acides nucléiques codent pour :
i) un antagoniste c-Jun, Jhdm1b, Id1, Glis1, Sall4, et Lrh1 ;
ii) un antagoniste c-Jun, Jhdm1b, Id1, Glis1, et Sall4 ;
iii) un antagoniste c-Jun, Jhdm1b, Id1, Glis1, et Lrh1 ; ou
iv) un antagoniste c-Jun, Jhdm1b, Id1, Sall4, et Lrh1.

3. Le procédé selon la revendication 1 ou 2, dans lequel la composition comprend un ou plusieurs vecteurs d'expression comprenant les acides nucléiques codant pour les polypeptides.

4. Le procédé selon la revendication 3, dans lequel l'un ou plusieurs vecteurs d'expression sont choisis dans le groupe constitué par les plasmides, les cosmides, les vecteurs viraux, et les chromosomes artificiels.

5. Le procédé selon la revendication 4, dans lequel les vecteurs viraux sont choisis dans le groupe constitué par les vecteurs rétroviraux, les vecteurs lentiviraux, les vecteurs adénoviraux, les vecteurs viraux adéno-associés, les vecteurs du virus simien 40, les vecteurs du papillomavirus bovin, le virus d'Epstein-Barr, les vecteurs du virus de l'herpès, les vecteurs du virus de la vaccine, les vecteurs du virus du sarcome murin de Harvey, les vecteurs du virus de la tumeur mammaire murine, et les vecteurs du virus du sarcome de Rous.

6. Le procédé selon l'une quelconque des revendications 1 à 5, dans lequel la cellule somatique de mammifère est choisie parmi une cellule somatique de souris ou une cellule somatique humaine.

7. Le procédé selon la revendication 6, dans lequel, la cellule somatique de souris ou la cellule somatique humaine sont choisies dans le groupe constitué par : le fibroblaste, la cellule mononucléaire dérivée de la moelle osseuse, la cellule de muscle squelettique, la cellule adipeuse, la cellule mononucléée du sang périphérique, le macrophage, la cellule souche neurale, la cellule hépatique, le kératinocyte, le kératinocyte oral, la cellule dermique du follicule pileux, la cellule épithéliale gastrique, la cellule épithéliale du poumon, la cellule synoviale, la cellule rénale, la cellule épithéliale de la peau et la cellule ostéoblaste.

8. Une composition pour favoriser la formation d'une cellule souche pluripotente induite, dans laquelle ladite composition comprend des acides nucléiques codant pour les polypeptides suivants : un antagoniste c-Jun, Jhdm1b, Id1, et au moins un facteur choisi uniquement parmi Glis1, Sall4 et Lrh1 ;
dans lequel l'antagoniste c-Jun comprend :
c-JunDN ayant la séquence d'acides aminés de SEQ ID NO. 2 ou une variante fonctionnelle de celle-ci, dans laquelle la variante fonctionnelle de celle-ci a un domaine bZIP mais manque d'un domaine de transactivation et présente au moins 70 % d'homologie avec SEQ ID NO. 2, ou
Jdp2 ayant la séquence d'acides aminés de SEQ ID NO. 3 ou une variante fonctionnelle de celle-ci, dans laquelle la variante fonctionnelle de celle-ci a un domaine bZIP mais manque d'un domaine de transactivation et présente au moins 70 % d'homologie avec SEQ ID NO. 3, et
dans laquelle, lorsque l'antagoniste c-Jun est c-JunDN, ladite composition ne comprend pas d'acides nucléiques codant pour les quatre groupes de polypeptides suivants : (1) Sox2, Klf4 et c-Myc, (2) Oct3/4, Klf4 et c-Myc, (3) Oct3/4, Lkf et Sox2, ou (4) Klf4 et Sox2.

9. La composition selon la revendication 8, dans laquelle les acides nucléiques codent pour :
i) un antagoniste c-Jun, Jhdm1b, Id1, Glis1, Sall4, et Lrh1 ;
ii) un antagoniste c-Jun, Jhdm1b, Id1, Glis1, et Sall4 ;
iii) un antagoniste c-Jun, Jhdm1b, Id1, Glis1, et Lrh1 ; ou
iv) un antagoniste c-Jun, Jhdm1b, Id1, Sall4, et Lrh1.

10. La composition selon la revendication 8 ou 9, dans laquelle la composition comprend un ou plusieurs vecteurs d'expression comprenant les gènes codant pour les polypeptides.

11. La composition selon la revendication 10, dans laquelle l'un ou plusieurs vecteurs d'expression sont choisis dans le groupe constitué par les plasmides, les cosmides, les vecteurs viraux, et les chromosomes artificiels.

12. La composition selon la revendication 11, dans laquelle les vecteurs viraux sont choisis dans le groupe constitué par les vecteurs rétroviraux, les vecteurs lentiviraux, les vecteurs adénoviraux, les vecteurs viraux adéno-associés, les vecteurs du virus simien 40, les vecteurs du papillomavirus bovin, le virus d'Epstein-Barr, les vecteurs du virus de l'herpès, les vecteurs du virus de la vaccine, les vecteurs du virus du sarcome murin de Harvey, les vecteurs du virus de la tumeur mammaire murine, et les vecteurs du virus du sarcome de Rous.

13. Utilisation de la composition selon l'une quelconque des revendications 8 à 12 dans la préparation d'une cellule souche pluripotente induite.
